Europäisches Patentamt

European Patent Office

Office européen des brevets

(19)

(11) Numéro de publication: **0 269 520**
**A2**

## (12) DEMANDE DE BREVET EUROPEEN

(21) Numéro de dépôt: 87402631.3

(22) Date de dépôt: 23.11.87

(51) Int. Cl.⁴: **C 12 N 7/00**
A 61 K 39/21, G 01 N 33/569,
C 07 K 15/00, C 12 N 5/00,
C 12 N 15/00, C 12 Q 1/70,
C 12 P 21/02

(30) Priorité: 21.11.86 US 933184
22.01.87 EP 87400151

(43) Date de publication de la demande:
01.06.88 Bulletin 88/22

(84) Etats contractants désignés:
AT BE CH DE ES FR GB GR IT LI LU NL SE

(71) Demandeur: INSTITUT PASTEUR
25-28, rue du Docteur Roux
F-75724 Paris Cédex 15 (FR)

(72) Inventeur: Montagnier, Luc
21 rue de Malabry
F-92350 Le Plessis-Robinson (FR)

Chamaret, Solange
324 rue Lecourbe
F-75015 Paris (FR)

Guétard, Denise
4B, rue Anselme Payen
F-75015 Paris (FR)

Alizon, Marc
71 rue du Cardinal Lemoine
F-75005 Paris (FR)

Clavel, François
83 rue de l'Assomption
F-75016 Paris (FR)

Guyader, Mireille
2 Square Rocamadoure
F-75016 Paris (FR)

Sonigo, Pierre
23 rue Gutenberg
F-75015 Paris (FR)

Brun-Vezinet, Françoise
24 boulevard Saint-Germain
F-75005 Paris (FR)

Rey, Marianne
10 rue du Temple
F-75004 Paris (FR)

Rouzioux, Christine
21 rue de Dantzig
F-75015 Paris (FR)

Katlama, Christine
8 rue de Jarente
F-75004 Paris (FR)

(74) Mandataire: Gutmann, Ernest et al
S.C. Ernest Gutmann - Yves Plasseraud 67, boulevard
Haussmann
F-75008 Paris (FR)

Le (Les) microorganisme(s) a (ont) été déposé(s) auprès Cultures Nationales de Microorganismes sous le(s) numéro(s) I-232, I-502, I-532, I-642, I-643, I-519, I-537, I-626, I-633, I-627, I-628.

(54) Rétrovirus du type HIV-2 susceptible de provoquer le sida, et ses constituants antigéniques et nucléiques.

(57) L'invention concerne une nouvelle variété de rétrovirus, dénommés HIV-2, dont des échantillons ont été déposés à l'ECACC sous les numéros 87.01.1001 et 87.01.1002 et à la NCIB sous les numéros 12.398 et 12.399. Elle concerne aussi les antigènes susceptibles d'être obtenus à partir de ce virus, en particulier des protéines p12, p16, p26 et gp140. Ces différents antigènes sont applicables au diagnostic de la maladie, notamment par mise en contact avec un sérum du malade chez lequel le diagnostic doit être effectué. Elle concerne des compositions immunogènes contenant plus particulièrement la glycoprotéine sp140. Elle concerne enfin des séquences nucléotidiques, utilisables notamment en tant que sondes d'hybridation, dérivées de l'ARN de HIV-2.

EP 0 269 520 A2

Bundesdruckerei Berlin

## Description

Rétrovirus du type HIV-2 susceptible de provoquer le SIDA, et ses constituants antigéniques et nucléiques.

L'invention est relative à une nouvelle classe de virus, ayant la capacité de provoquer des lymphadénopathies susceptibles d'être relayées ensuite par le syndrome d'immuno-déficence acquies (SIDA) chez l'homme. L'invention concerne également des antigènes susceptibles d'être reconnus par des anticorps induits chez l'homme par cette nouvelle classe de virus. Elle concerne également les anticorps induits par des antigènes obtenus à partir de ces virus.

Cette invention concerne, en outre, des séquences d'AND cloné soit présentant une analogie de séquence, soit une complémentarité avec l'ARN génomique du virus précité. Elle concerne aussi des procédés de préparation de ces séquences d'ADN cloné.

L'invention est également relative à des polypeptides contenant des séquences d'amino-acides codées par les séquences d'ADN cloné.

De plus, l'invention concerne des applications de ces antigènes au diagnostic in vitro chez l'homme de potentialités de certaines formes du SIDA et, en ce qui concerne certains d'entre eux, à la production de compositions immunogènes et de compositions vaccinantes contre ce rétrovirus. De même l'invention concerne les applications aux mêmes fins des susdits anticorps et, pour certains d'entre eux, leur application à la production de principes actifs de médicaments contre ces SIDAS humains.

L'invention concerne enfin l'application des sé quences d'ADN cloné et de polypeptides obtenus à partir de ces séquences comme sondes dans des kits de diagnostic.

L'isolement et la caractérisation d'un premier rétrovirus, dénommé LAV, dont avait été reconnue la responsabilité dans le développement du SIDA, a fait l'objet d'une description dans un article de F. BARRESINOUSSI et al dès 1983 (Science, vol. 220, no 45-99, 20, p. 868-871. L'application au diagnostic de la présence d'anticorps contre le virus de certains extraits de ce dernier, et plus pariculièrement de certaines de ses protéines, à été plus spécialement décrite dans la demande de brevet européen no 138.667. Depuis, d'autres souches similaires et des variants du LAV ont été isolés. On rappellera pour mémoire ceux connus sous les désignations HTLV-III et ARV.

En application des nouvelles régles de nomenclature publiées dans Nature, en mai 1986, les rétrovirus susceptibles d'induire chez l'homme les susdites lymphadenopaties et le SIDA, seront désignés globalement par "HIV", abréviation de l'expression anglaise "Human immunodeficiency Virus" (ou virus d'immunodéficience humaine). Le sous-ensemble des rétrovirus formés par LAV et de ces variants a initialement été désigné par les expressions "LAV type I" ou "LAV-I". Le même sous-ensemble sera désigné ci-après par la désignation HIV-1, étant entendu que l'expression LAV sera encore conservée pour désigner celle parmi les souches de rétrovirus (notamment LAAV, IDAV-1 et IDAV-2) appartenant à la classe des virus HIV-1 décrite dans la susdite demande de brevet européen 138.667, qui a été utilisée dans les essais comparatifs décrits plus loin, à savoir LAV$_{BRU}$, qui a été déposée à la Collection des Cultures Nationales de Microorganismes (CNCM) de l'Institut Pasteur de Paris, France, le 15 Juillet 1983, sous le no I-232.

Le nouveau rétrovirus faisant l'objet du présent brevet et les souches de virus qui en sont proches et qui, comme lui sont susceptibles de se multiplier dans des lymphocytes humains, antérieurement appelées "LAV type II" ou "LAV II" sont désormais appelées "HIV-2", étant entendu que les désignations de certains isolats de HIV-2 décrits plus loin seront suivies des trois lettres faisant référence aux malades à partir desquels ils ont été isolés.

On peut définir l'ensemble "HIV-2" comme un ensemble de virus ayant in vitro un tropisme pour des lymphocytes T4 humains, et qui ont un effet cytopathogène à l'égard de ces lymphocytes, lorsqu'ils s'y multiplient, pour alors causer soit des poly-adénopathies généralisées et persistantes, soit un SIDA. Les rétrovirus HIV-2 se sont révélés distincts des virus de type HIV-1 dans les conditions évoquées plus loin. Comme ces derniers, ils sont distincts des autres rétrovirus humains déjà connus (HTLV-I et HTLV-II).

Bien qu'il y ait une assez grande variabilité génétique du virus, les différentes souches HIV-1 isolées à ce jour, à partir des malades américains, européens, haï tiens et africains ont en commun des sites antigéniques conservés sur leurs principales protéines : protéine du noyau (core) p25 et glycoprotéine d'enveloppe gp110 et protéine transmembranaire gp41-43. Cette parenté permet par exemple à la souche prototype LAV d'être utilisée comme souche d'antigènes pour la détection d'anticorps contre tous les virus de la classe HIV-1, chez toutes les personnes qui les portent, quelle que soit leur origine. Cette souche est donc utilisée actuellement pour la détection d'anticorps anti-HIV-1 chez les donneurs de sang et les maledes par les techniques d'immunofluorescence, notamment par les techniques dites ELISA, "Western Blot" (ou immuno-empreintes) et "RIPA" abréviation anglaise de "Radio Immunoprecipitation Assay" (test de radio-immunoprécipitation).

Cependant dans une étude sérologique effectuée avec un lysat de HIV-1 sur des malades originaires de l'Afrique de l'Ouest, il a été observé que certains d'entre eux donnaient des réactions séro-négatives ou très faiblement positives, alors qu'ils présentaient les signes cliniques et immunologiques du SIDA.

C'est à partir des lymphocytes mis en culture de l'un de ces malades, que l'on a isolé un premier rétrovirus HIV-2, dont la structure en microscopie électronique et le profil des protéines en gel d'électrophorèse SDS ont une similitude avec ceux de HIV-1. Mais ce nouveau rétrovirus HIV-2 ne présente globalement qu'une faible parenté avec HIV-1, tant du point de vue de l'homologie antigénique de ses protéines que de l'homologie de son matériel génétique.

Ce nouveau rétrovirus ou des rétrovirus ayant des propriétés antigéniques et immunologiques équivalentes, peuvent donc constituer des sources d'antigènes pour le diagnostic de l'infection par ce virus et des variants qui induisent un SIDA, du type de celui que l'on avait observé pour les premières fois chez des malades africains ou des personnes ayant séjourné en Afrique.

Le premier isolement de ce virus a été réalisé à partir du sang, prélevé sous héparine, d'un malade de 28 ans, hétérosexuel, qui n'avait jamais été transfusé et qui n'était pas toxicomane. Il présentait depuis 1983 une importante diarrhée chronique, un amaigrissement important (17 kg) avec de la fièvre par intermittence. Plus récemment il avait présenté des infections à Candida et Serratia, dont une candidose oesophagienne, typique du SIDA.

Ce patient présentait également une anémie, une anergie cutanée, une lymphopénie, un rapport lymphocytes T4/lymphocytes T8 de 0,15 avec un taux en lymphocytes T4 inférieur à 100 par mm³ de sérum. Ses lymphocytes en culture ne répondaient pas à la stimulation par la phytohémoglutinine et la concanavaline A. On a également diagnostiqué chez ce malade des bactériémies récurrentes dues à S. enteriditis, des cryptosporidioses, des infections dues à Isospora belli et des toxoplasmoses cérébrales.

L'ensemble de ces signes témoignait des symptômes "complexes liés au SIDA" ou "ARC" (abréviation anglaise de "AIDS Related Complex"), du type de ceux causés par le virus HIV-1. Ces différentes observations étaient également conformes aux critères apliqués par le Centre de Contrôle des Maladies (Center of Disease Control ou CDC) d'Atlanta, Etats-Unis.

La mise en culture des lymphocytes de ces malades et l'isolement du rétrovirus ont été effectués selon la technique déjà décrite pour l'isolement du HIV-1 (1) dans l'article de BARRE-SINOUSSI et Coll. et la demande de brevet européen no 84 401834/0 138 667. On les rappelle brièvement ci-après. Des lymphocytes stimulés pendant 3 jours par de la phytohémagglutinine (PHA) ont été cultivés en milieu de culture RPM1-16-40 additionné de 10 % de sérum de veau foetal et de $10^{-5}$M β-mercaptoéthanol, d'interleukine-2 et de sérum anti-interféron α humain.

La production de virus a été suivie par son activité transcriptase inverse. Dans le surnageant de culture, le pic d'activité virale est apparu entre le 14ème et le 22ème jour, puis a diminué. Le déclin et la mort de la culture cellulaire ont suivi. Comme avec le HIV-1, des coupes de lymphocytes infectés par HIV-2 révèlent en microscopie électronique des virions parvenus à maturité et des particules virales bourgeonnant à la surface des cellules infectées. Les lignées cellulaires utilisées pour réaliser les cultures de ces virus isolés peuvent être selon les cas, les lignées cellulaires du type HUT, CEM, MOLT ou de toute lignée lymphocytaire immortalisée portant dès récepteurs T4.

Le virus a ensuite été propagé sur des cultures de lymphocytes de donneurs de sang, puis sur des lignées continues d'origine leucémique, telles que HUT 78. Il a été caractérisé comme étant sensiblement distinct du HIV-I par ses antigènes et son acide nucléique. Le virus a été purifié comme décrit dans les documents antérieurs déjà mentionnés. Un premier isolat de ce virus a été déposé à la C.N.C.M. le 19 décembre 1985, sous le no I-502. Il a été désigné postérieurement sous le nom LAV-II MIR. Un second isolat a été déposé à la C.N.C.M le 21 février 1986 sous le no I-532. Ce second isolat a pour nom de référence LAV-II ROD. Il sera quelquefois fait simplement référence plus loin, à MIR ou à ROD.

D'une façon générale l'invention concerne tout variant des virus précédents ou tout virus équivalent (par exemple tel que HIV-2 IRMO et HIV-2 EHO déposés à la CNCM le 19 Décembre 1986 sous les numéros I-642 et I-643 respectivement) contenant des protéines de structure qui présentent les mêmes propriétés immunologiques que celles des virus HIV-2 déposés à la CNCM sous les numéros I-502, ou I-532. Il sera encore revenu plus loin sur les définitions critères d'équivalence.

L'invention concerne encore un procédé de production du virus HIV-2 ou de variants de celui-ci dans les lignées cellulaires permanentes dérivées de lymphocytes T4, ou portant le phénotype T4, ce procédé consistant à cultiver ces lignées préalablement infectées avec le virus HIV-2, et notamment lorsque le niveau d'activité de la transcriptase inverse (ou reverse-transcriptase) a atteint un seuil déterminé, à récupérer les quantités de virus libérées dans le milieu de culture.

Une lignée permanente préférée en vue de la culture de HIV-2 est, par exemple, du type cellules HUT 78. Une lignée de HUT 78, infectée par HIV-2, a été déposée le 6 février 1986 à la CNCM sous le no I-519. La culture est, par exemple, réalisée comme suit :

Les cellules HUT 78 ($10^6$/ml) sont mises en cocultures avec des lymphocytes humains normaux infectés ($10^6$/ml). Le milieu de culture est du RPMI 1640 avec 10% de sérum de veau foetal. Au bout de 15 à 21 jours, on observe un effet cytopathogène des cellules HUT 78. On dose la reverse transcriptase une semaine après cette observation, dans le surnageant de culture. On peut alors commencer à récupérer le virus à partir de ce surnageant.

Une autre lignée préférée pour la culture appartient aux lignées des cellules connues sous la désignation CEM.

L'infection, puis la culture des cellules CEM infectées peuvent notamment être réalisées comme suit.

On procède à la coculture de lymphocytes T4 préalablement infectés avec le virus HIV-II et de cellules non infectées de la lignée CEM pendant le temps nécessaire à l'infection des CEM. On poursuit d'ailleurs ensuite les conditions de culture dans un milieu approprié, par exemple celui décrit ci-après et, lorsque l'activité transcriptase inverse des cellules infectées a atteint un niveau suffisant, on récupère le virus produit à partir du milieu de culture.

On a notamment réalisé une co-culture dans les conditions précisées ci-après de lymphocytes T4 humains qui avaient été infectés cinq jours auparavant avec une souche de virus HIV-II provenant d'un patient désigné ci-après "ROD", d'une part, et de CEM, d'autre part.

Les lymphocytes T4 infectés, préalablement activés par la phytohémaglutinine, se sont révélés posséder une activité de transcriptase inverse de 5 000 cpm/$10^6$ lymphocytes T normaux trois jours après le début de l'infection. La culture a été poursuivie jusqu'à ce que l'activité transcriptase inverse mesurée ait atteint 100 000 cpm dans le surnageant. Ces lymphocytes T4 ont alors été mis en contact avec les cellules CEM (3. $10^6$ lymphocytes T normaux infectés) et incubées dans le milieu de culture suivant : RPMI 1640 contenant 2,92 mg/ml de L glutamine, 10 % de sérum de veaux foetal décomplémenté, 2µg/ml de polybrène, 0,05 % de sérum anti-interféronalpha, 100 000 µg/ml de pénicilline, 10 µg/ml de streptomycine et 10 000 µg/ml de néomycine.

Le milieu de culture est changé deux fois par semaine.

Les mesures d'activité transcriptase inverse mesurée dans le surnageant ont été les suivantes :
- au jour 0 :   1 000 (bruit de fond)
- au jour 15 :    20 000
- au jour 21 :   200 000
- au jour 35 :   1 000 000

Une culture CEM infectée par le virus HIV-2 a été déposée à la Collection Nationale de Culture de Micro-organismes de l'Institut Pasteur (C.N.C.M) sous le n° I-537 le 24 mars 1986.

Quelques caractéristiques des antigènes et des acides nucléiques, entrant dans la constitution de HIV-2, résultent des essais réalisés dans les conditions indiquées ci-après. Elles seront souvent mieux appréciées par comparaison avec des caractéristiques du même type se rapportant à d'autres types de rétrovirus, notamment HIV-1 et SIV.

Il sera fait référence dans ce qui suit aux dessins dans lesquels :

- les figures 1a, 1b et 1c se rapportent à des essais d'immunoprécipitation croisée entre des sérums respectivement de patients infectés par HIV-1 et HIV-2, et de macaques rhésus infectés avec STLV-III, d'une part, et des extraits viraux de HIV-1, d'autre part ;

- les figures 2a et 2b présentent des résultats comparatifs quant aux mobilités électrophorétiques des protéines de HIV-1, et HIV-2 et STLV-III respectivement, dans des gels de polyacrylamide-SDS ;

- la figure 3 présente des résultats d'hybridation croisée entre des séquences génomiques de HIV-1, HIV-2 et STLV-III, d'une part, et des sondes contenant différentes séquences subgénomiques du virus HIV-1, d'autre part.

- la figure 4 est une carte de restriction de l'ADNc dérivé de l'ARN de HIV-2 ROD ;

- la figure 5 est une carte de restriction d'un fragment E2 de l'ADNc dérivé de HIV-2, ce fragment contenant une région correspondant à la région LTR 3′ de HIV-2 ;

- la figure 6 est la séquence nucléotidique d'une partie de E2, cette séquence correspondant à la région U3/R de HIV-2 ;

- la figure 7 fait apparaître :
- d'une part et schématiquement, des éléments de structure de HIV-1 (figure 5A) et, alignée avec une région contenant le LTR 3′ de HIV-1, la séquence dérivée de la région E2 de l'ADNc de HIV-2 et,
- d'autre part, les mises en alignement au prix d'un certain nombre de délétions et d'insertions, des nucléotides communs, contenus respectivement dans la séquence dérivée de la région E2 de HIV-2, et dans la séquence correspondante de HIV-1 (fig. 5 B)

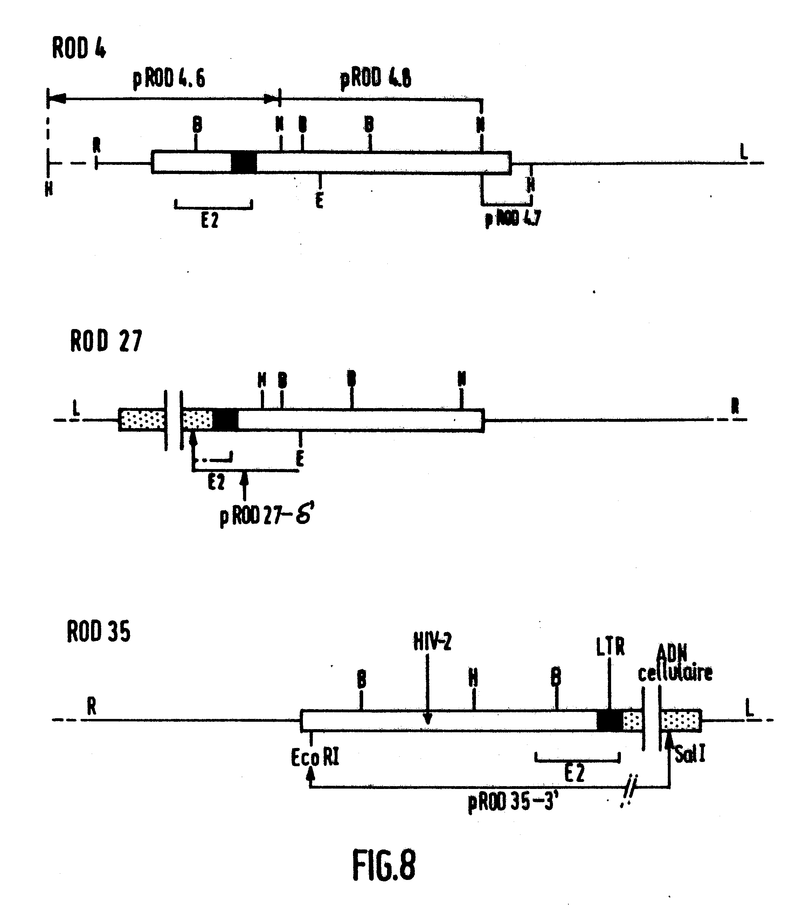

- la figure 8 montre schématiquement les structures de plusieurs clones d'un phage λ modifié par divers insérats issus de l'ADNc dérivé d'HIV-2 (clones ROD4, ROD27 et ROD35) ; on y a également représenté schématiquement des séquences dérivées à leur tour de ROD4, ROD27 et ROD35, sous-clonées dans un plasmide pvc 18, ces dernières séquences étant placées en correspondance avec les régions de ROD4, ROD27 et ROD35 dont elles sont respectivement issues.

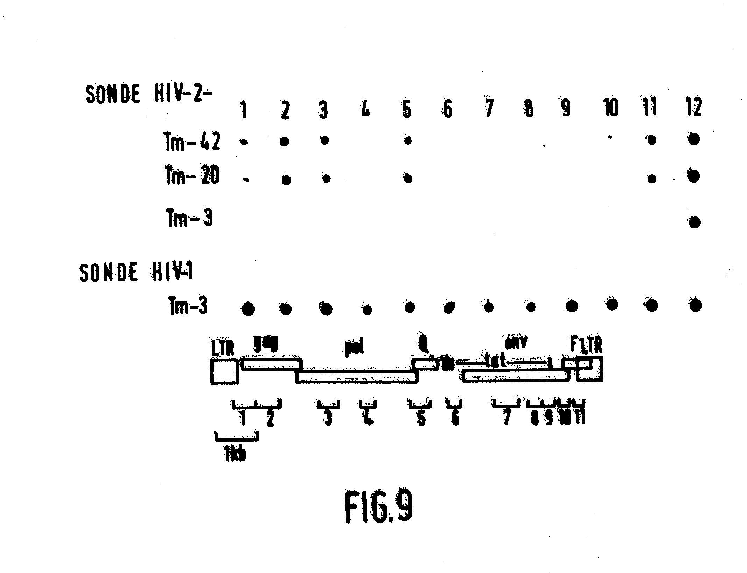

- la figure 9 fait apparaître les intensités relatives d'hybridation entre
a/ onze fragments prélevés à partir de différentes régions du génome complet de HIV-1 (schématiquement représentés dans la partie inférieure de la figure d'une part, et l'ADNc de HIV-2, contenu dans ROD4, d'autre part, et
b/ des fragments issus de HIV-II avec le même ADNc.

D'une façon générale, les antigènes de HIV-2 utilisés dans les tests comparatifs dont la description suit sont issus de la souche de HIV-2 MIR déposée à la C.N.C.M. sous le n° I-502, et les séquences d'ADN dérivés de l'ADN génomique de HIV-2 sont issus de la souche HIV-2 ROD déposée à la C.N.C.M. sous le n° I-532.

I - ANTIGENES, NOTAMMENT PROTEINES ET GLYCOPROTEINES

Le virus initialement cultivé dans HUT 78 a été marqué métaboliquement par la $^{35}$S- cystéine et la $^{35}$S-méthionine, les cellules infectées étant incubées en présence de ces acides aminés radioactifs en milieu de culture dépourvu de l'acide aminé non marqué correspondant, pour une période de 14 à 16 heures, en particulier selon la technique décrite dans l'article répertorié en [21] dans la bibliographie présentée à la fin de la description, pour ce qui est du marquage à la $^{35}$S- cystéine. Le surnageant est ensuite clarifié, puis le virus ultracentrifugé une heure à 100 000 g sur un coussin de 20 % de saccharose. Les principaux antigènes du virus sont séparés par électrophorèse dans un gel de polyacrylamide (12,5 %), dans des conditions dénaturantes (SDS) ou dans un gel de polyacrylamide (10 %) + bisacrylamide (0,13 % ) avec SDS (0,1 % en concentration finale). On utilise comme poids moléculaire de référence les marqueurs colorés suivants

BRL :

| | | |
|---|---|---|
| myosine : | 200 | Kd |
| phosphorylase B : | 97,4 | Kd |
| BSA : | 68 . | Kd |
| ovalbumine : | 43 | Kd |
| α chymotrypsine : | 25,7 | Kd |
| β lactoglobuline : | 18,4 | Kd |
| lysozyme : | 14,3 | Kd |

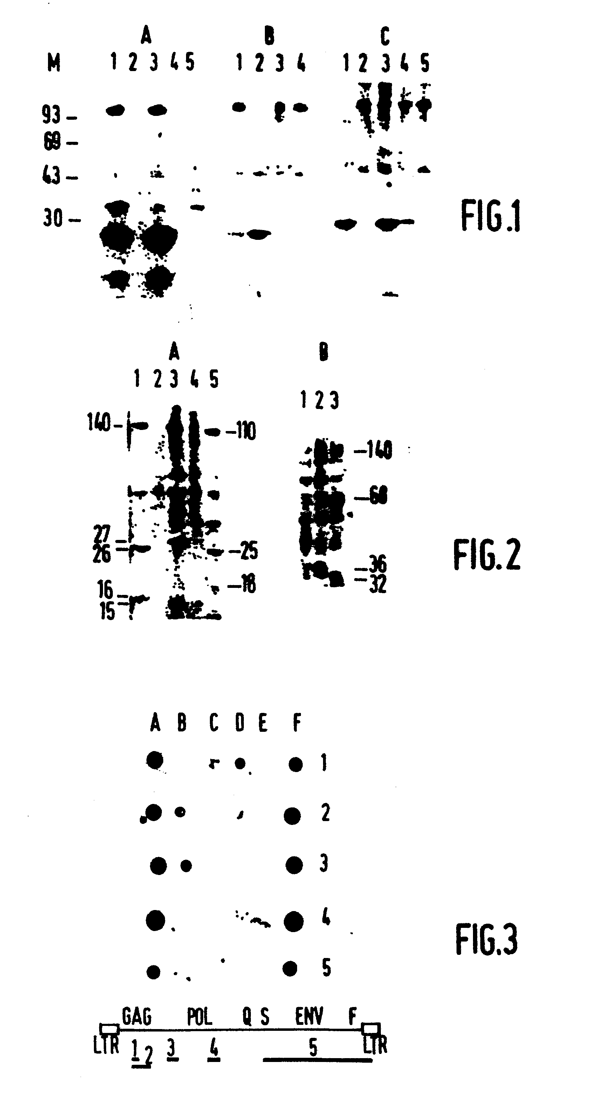

D'autres marqueurs de poids moléculaires ont été utilisés dans d'autres essais. Il est en particulier fait référence aux figures 1a, 1b et 1c qui renvoient à d'autres marqueurs de poids moléculaires connus (sous la lettre M. dans ces figures). Les antigènes sont encore mieux distingués après immunoprécipitation (RIPA) ou par immunoempreintes (Western blot) en utilisant les anticorps présents dans le sérum du malade : leurs poids moléculaires apparents déterminés par leurs migrations apparentes sont très proches de ceux des antigènes du HIV-1.

D'une façon générale il est précisé que dans ce qui suit les nombres qui suivaient les indications "p" et/ou "gp" correspondent aux poids moléculaires approximatifs des protéines et/ou glycoprotéines correspondantes divisés par 1000. Par exemple la p36 a un poids moléculaire de l'ordre de 36 000. Il est cependant entendu que ces valeurs de poids moléculaires peuvent varier dans un intervalle pouvant atteindre 5%, voire 10% ou même plus, selon les techniques utilisées pour les déterminations de ces poids moléculaires.

La répétition des essais a permis de mieux cerner les poids moléculaires apparents des antigènes de HIV-2. Ainsi on a constaté que les poids moléculaires des trois protéines du noyau (core), auxquelles on avait initialement attribué des poids moléculaires de l'ordre de 13.000, 18.000 et 25.000 respectivement, avaient en fait des poids moléculaires apparents plus proches des valeurs suivantes : 12.000, 16.000 et 26.000, respectivement. Ces protéines sont ci-après respectivement désignées par les abréviations p12, p16 et p26.

De même l'existence de bandes protéine ou glycoprotéine dont les poids moléculaires apparents ont été appréciés à des valeurs qui pouvaient s'étager de 32.000 à 42.000-45.000. La répétition des mesures a finalement permis de préciser une bande correspondant à un poids moléculaire apparent de 36.000. Dans ce qui suit, cette bande est désignée par l'abréviation p36. Une autre bande à 42.000-45.000 (p42) est constamment observée aussi. L'une ou l'autre des p36 ou p42 constitue vraisemblablement une glycoprotéine transmembranaire du virus.

Une glycoprotéine majeure d'enveloppe ayant un poids moléculaire de l'ordre de 130-140 Kd est constamment observée : cette glycoprotéine est ci-après désignée par l'expression gp140. Il convient de noter que, d'une façon générale, les poids moléculaires sont appréciés avec une précision de plus ou moins 5 %, cette précision pouvant même devenir un peu moindre pour les antigènes de haut poids moléculaire, comme on l'a constaté pour la gp140 (poids moléculaire de 140 Kd ± 10 %). L'ensemble de ces antigènes sont peu ou pas reconnus (lorsqu'ils sont marqués par la $^{35}$S-cystéine) par des sérums de malades contenant des anticorps anti-HIV-1 dans les systèmes de détection utilisés au laboratoire ou par utilisation des tests mettant en oeuvre des lysats de HIV-1, tels que ceux commercialisés par DIAGNOSTICS PASTEUR sous la marque "ELAVIA". Suele la protéine p26 a été faiblement immunoprécipitée par de tels sérums. La protéine d'enveloppe ne l'a pas été. Le sérum du malade infecté par le nouveau virus (HIV-2) reconnaît faiblement une protéine p34 du HIV-1. Dans le système de détection utilisé, les autres protéines du HIV-1 n'ont pas été reconnues.

En revanche le HIV-2 possède certaines protéines présentant une certaine parenté immunologique avec des protéines ou glycoprotéines structurales semblables, séparables dans des conditions analogues, à partir d'un rétrovirus récemment isolé à partir de singes macaques rhésus captifs, de l'espèce des macaques rhésus, alors que cette parenté immunologique tend à s'effacer pour d'autres protéines ou glycoprotéines. Ce dernier rétrovirus, qui est présumé être l'agent étiologique de SIDAs chez les singes, a été désigné par les chercheurs qui l'ont isolé (références bibliographiques (16-18) ci-après) sous l'appellation "STLV-III mac". Pour la commodité du langage, il ne sera plus désigné dans ce qui suit que par l'expression "STLV-III" (ou encore par l'expression SIV, abréviation anglaise de "Simian Immunodeficiency Virus" (virus d'immunodéficience du singe)).

Un autre rétrovirus, désigné "STLV-III AGM", (ou SIV AGM) a été isolé chez des singes verts sauvages. Mais, contrairement au virus présent chez le singe macaque rhésus, la présence de "STLV-III$_{AGM}$" ne semble pas induire une maladie du type SIDA chez le singe vert d'Afrique.

Toutefois, la parenté immunologique des protéines et des glycoprotéines structurales, et par conséquent la parenté de leurs séquences nucléiques, de HIV-II d'une part, et des rétrovirus STLV-III$_{mac}$ et STLV-III$_{AGM}$ d'autre part, reste limitée. Des expériences ont permis d'établir une première distinction entre les rétrovirus capables d'infecter l'homme ou le singe, il en ressort ceci :

- le virus HIV-II ne se multiplie pas de façon chronique dans les lymphocytes du singe macaque rhésus, lorsqu'il a été injecté in vivo et dans des conditions opératoires permettant le développement du virus STLV-III$_{mac}$ telles qu'elles ont été décrites par N.L. Letvin et al, Science (1985)vol 230, 71-75.

Cette apparente inaptitude de HIV-2 à se développer chez le singe dans conditions naturelles, permet de différencier biologiquement le virus HIV-II d'une part, et les isolats du virus STLV-III, d'autre part.

En mettant en oeuvre les mêmes techniques que celles rappelées plus haut, il a été constaté que l'on pouvait également obtenir à partir de STLV-III :

- une protéine principale du noyau p27, ayant un poids moléculaire de l'ordre de 27 kilodaltons,
- une glycoprotéine majeure d'enveloppe, gp140,
- une protéine vraisemblablement transmembranaire p32, qui n'est pas observée en RIPA lorsque le virus a au préalable été marqué par la $^{35}$S-cystéine, mais qui peut être observée dans les essais d'immunoempreintes (Western blots), sous forme de bandes larges.

La glycoprotéine majeure d'enveloppe de HIV-2 s'est révélée être plus proche immunologiquement de la glycoprotéine majeure d'enveloppe de STLV-III que de la glycoprotéine majeure d'enveloppe de HIV-1.

Ces cosntatations s'imposent non seulement au niveau des poids moléculaires : 130-140 kilodaltons pour les glycoprotéines majeures de HIV-2 et de STLV-III, contre environ 110 pour la glycoprotéine majeure d'enveloppe de HIV-1, mais aussi au niveau des propriétés immunologiques, puisque des sérums prélevés à partir de malades infectés par HIV-2, et plus particulièrement des anticorps formés contre la gp140 de HIV-2 reconnaissent la gp140 de STLV-III, alors que dans des essais semblables les mêmes sérums et les mêmes anticorps de HIV-2 ne reconnaissent pas la gp110 de HIV-1. Mais les sérums anti-HIV-1 qui n'ont jamais réagi avec la gp140 de HIV-2 précipitent une protéine de 26 Kdal marquée par la $^{35}$S-cystéine, contenue dans les extraits de HIV-II.

La protéine majeure du noyau (core) de HIV-2 semble présenter un poids moléculaire moyen (environ 26.000) intermédiaire entre celui de la p25 de HIV-1 et la p27 de STLV-III.

Ces observations résultent des essais réalisés avec des extraits viraux obtenus à partir du HIV-2 isolé à partir de l'un des patients susmentionnés. Des résultats similaires ont été obtenus avec des extraits viraux du HIV-2 isolé à partir du second patient.

Des cellules infectées respectivement avec HIV-1, HIV-2 et STLV-III ont été incubées dans un milieu contenant 200 μCi/ml de $^{35}$S-cystéine dans un milieu exempt de cystéine non marquée pendant 16 heures. Les surnageants clarifiés ont été centrifugés à 60.000 g pendant 90 minutes. Les culots ont été lysés dans un tampon RIPA(1), immunoprécipités avec différents sérums, puis soumis à une électrophorèse sur gel de polyacrylamide chargé en dodécylsulfate de sodium (SDS-PAGE).

Les résultats observés sont illustrés par les figures 1a, 1b et 1c.

Dans la figure 1a, sont présentés les résultats d'immunoprécipitation observés entre un extrait viral de HIV-1 obtenu à partir d'une lignée cellulaire CEM C1.13 et les sérums respectifs suivants :

- sérum positif anti-HIV-1 (bande 1),
- sérum obtenu du premier patient mentionné plus haut (bande 2),
- sérum d'un porteur africain sain d'anticorps anti-HIV-1 (bande 3),
- sérum obtenu à partir d'un macaque infecté avec STLV-III (bande 4) et
- sérum du deuxième patient susmentionné (bande 5).

Dans la figure 1b, sont rapportés les résultats d'immunoprécipitation observés entre les antigènes de HIV-2 obtenus à partir du premier patient, après culture préalable sur des cellules HUT-78, et différents sérums, plus particulièrement le sérum du premier patient sus-mentionné (bande 1), le sérum positif anti-HIV-1 (bande 2), le sérum du macaque infecté par STLV-III (bande 3), le sérum du second patient susdit (bande 4).

Enfin, la figure 1c illustre les résultats d'immunoprécipitation observés entre les antigènes d'un isolat de STLV-III otenu à partir d'un macaque présentant un SIDA de singe. Les sérums utilisés et auxquelles se réfèrent les bandes 1 à 5 sont les mêmes que ceux rappelés plus haut en rapport avec la figure 1a.

M se réfère aux marqueurs myosine (200 Kd) galactosidase (130 Kd), sérum albumune bovine (69 Kd), phosphorylase B (93 Kd), ovalbumine (46 Kd) et carbonate anhydrase (30 Kd).

Les figures 2a et 2b présentent les résultats comparatifs quant aux mobilités électrophorétiques des protéines de HIV-1, HIV-2 et STLV-III.

La figure 2a se rapporte aux essais réalisés avec des extraits de virus marqués par la $^{35}$S-cystéine, après immunoprécipitation sur SDS-PAGE. Les différentes bandes se rapportent aux extraits de virus suivants : virus obtenu à partir du patient 1 et immunoprécipité par le sérum provenant du même patient (bande 1), extrait du même virus immunoprécipité avec un sérum témoin négatif provenant d'une personne ne portant pas d'anticorps anti-HIV-1 ou anti-HIV-2 (bande 2), extrait de virus STLV-III immunoprécipité par un sérum provenant d'un macaque infecté par STLV-III (bande 3), immunoprécipitations observées entre des extraits du même virus et d'un sérum témoin négatif (bande 4), extrait de HIV-1 immunoprécipité par un sérum d'un patient européen infecté par le SIDA.

La figure 1b présente les résultats obtenus dans des essais de Western-blot (immunoempreinte). Des lysats cellulaires provenant de cellules HUT)78 non infectées ou infectées ont été soumis à une électrophorèse sur SDS-PAGE, puis transférés électrophorétiquement sur un filtre de nitrocellulose, avant d'être mis a réagir avec le sérum du premier patient susmentionné (sérum dilué au 1/100e). Le filtre de nitrocellulose a ensuite été lavé et la détection des anticorps fixés révélée avec des IgG anti-humaines de chèvre marquées par [125]I.

Les taches observées dans des bandes 1, 2 et 3 se rapportent respectivement aux essais d'agglutination entre le susdit sérum et des extraits de cellules HUT-78 non infectées (bande 1), des extraits de cellules HUT-78 infectées avec un virus HIV-2 (bande 2) et des extraits de cellules HUT-78 infectées par STLV-III (bande 3). Les nombres qui apparaissent dans les marges de chacune des bandes correspondent aux poids moléculaires approximatifs des protéines virales les plus représentatives (poids moléculaires en kilodaltons).

## II - ACIDES NUCLEIQUES

### 1) les ARNs du rétrovirus HIV-2

L'ARN du virus, déposé sur filtre d'après la technique du "spot blot" n'a pas hybridé, dans des conditions stringentes avec l'ADN du HIV-1.

Par "conditions stringentes" on entend : les conditions dans lesquelles la réaction d'hybridation entre l'ARN du HIV-2 et la sonde choisie marquée radioactivement au $p^{32}$ (ou marquée de façon différente), puis les lavages de la sonde sont faits. L'ybridation, sur membrane, est faite à 42°C en présence d'une solution aqueuse à 50% (volume/volume) de 0,1% SDS/5x55c, notamment formamide pendant 18 heures. La membrane sur laquelle a été faite la réaction d'hybridation est lavée ensuite à 65°C dans un tampon contenant 0,1 % de SDS et 0,1 X SSC.

Par "conditions non stringentes", on entend les conditions dans lesquelles la réaction d'hybridation et les lavages sont faits. L'hybridation est réalisée en mettant en contact avec la sonde choisie marquée au $p^{32}$ (ou marquée autrement), à savoir à 42°C dans un tampon 5 x SSc, 0,1% SDS contenant 30 % de formamide pendant 18 heures. Le lavage de la membrane est réalisé à 50°C avec un tampon contenant 0,1 % de SDS et 2 X SSC.

On a également réalisé des essais d'hybridation avec une sonde d'hybridation constituée par un plasmide recombinant pBT1 obtenu par clonage de l'ADN du HIV-1 provenant de λJ19 (Cell 1985, vol. 40, p.9) dans le vecteur pUC18. En conditions non stringentes, on n'a observé qu'une très faible gybridation entre l'ARN de HIV-2 et l'ADN cloné dérivé de HIV-1.

D'autres sondes contenant des séquences clonées de HIV-1 ont été utilisées :

a) Des sondes simple brin de l'ADN du HIV-1 subgénomique élaborées à partir de sous-clones du génome du HIV-1 et insérées dans le phage M13. Les régions clonées concernaient le gène protéase ou le gène "endonucléase".

Seule une sonde de la région endonucléase de HIV-1 (séquence de nucléotides comprise entre les bases no 3760 et 4130) a donné une hybridation faible en conditions non stringentes avec le HIV-2. La sonde "protéase" (séquence de nucléotides de HIV-1 comprise entre les bases no 1680 et 1804) n'a pas hybridé, même en codnitions non stringentes avec le HIV-2.

b) Une sonde pRS3 constituée par la séquence codant pour la région "enveloppe" du HIV-1 (sous-clonage dans pUC18) n'a pas donné d'hybridation en conditions non stringentes avec HIV-2.

La technique du "spot blot"est appelée aussi "dot blot" (transfert par taches).

Des résultats d'hybridation supplémentaires entre des ARNs génomiques de HIV-1, HIV-2 et STLV-III, d'une part, et des sondes contenant différeentes séquences sub-génomiques du virus HIV-1, d'autre part, apparaissent dans la figure 3.

Les surnageants des différents milieux de culture (à raison de 0,5 à 1 ml pour chaque tache) ont été centrifugés pendant 5 minutes à 45.000 tours par minute ; les culots ont été remis en suspension dans un tampon NTE contenant 0,1 % de SDS et déposés sur filtre de nitrocellulose. Celui-ci a été prétrempé dans un milieu 2 x SSC (NaCl 0,3 M, citrate de sodium 0,03 M). Après cuisson (pendant 2 heures à 80°C), les filtres ont été hybridés avec diverses sondes contenant des sous-fragments génomiques de HIV-1, dans des conditions non stringentes (30 % de formamide, 5 x SSC, 40°C), lavés avec une solution 2 x SSC et contenant 0,1 % de SDS, à 50°C, puis autoradiographiés pendant 48 heures à -70°C avec des écrans d'amplification.

Les sondes 1-4 sont des sondes à brin unique obtenues par la méthode de "coupe principale" (prime cut method) telle que décrite en (25). En bref des fragments mono-brins issus du virus M13 et portant des insérats de HIV-I subgénomiques (30) ont été ligaturés à des fragments d'oligomères (17 nucléotides) issus de M13 (BIOLABS). Le brin complémentaire a été ensuite synthétisé avec l'enzyme de Klenow dans un tampon TM (Tris 10 mM, pH 7,5, $MgCl_2$, 10 mM) en présence de dATP, dGTP, dTTP et dCTB marqués par du $^{32}p$ en alpha (Amersham, 3000 Ci/mMol). L'ADN a ensuite été digéré par les enzymes de restriction appropriés, dénaturé par la chaleur et soumis à électrophorèse sur un gel dénaturant de polyacrylamide (contenant 6 % d'acrylamide, de l'urée 8 M dans un tampon TDE). Le gel a ensuite été autoradiographié pendant 5 minutes. La sonde a ensuite été découpée et éluée dans un tampon de NaCl 300 mM, SDS 0,1 %. Des activités spécifiques (AS) de ces sondes à brin unique ont été estimées à $5.10^8$-$10^9$ désintégrations par minute/ microgramme (dpm/μg).

Les séquences caractéristiques contenues dans les différentes sondes étaient les suivantes :

sonde 1 : nucléotides 990-1070,
sonde 2 : nucléotides 980-1260,
sonde 3 : nucléotides 2170-2240,
sonde 4 : nucléotides 3370-3640.

Les numérotations des nucléotides qui précèdent sont celles envisagées dans l'article référencé en (30).

Enfin la sonde 5 consiste en un plasmide pUC-18 portant le fragment EcoR1-Sac1 du HIV cloné dans λJ19 (31), lequel a fait l'objet d'une translation de coupure (nick-translation) pour obtenir un AS d'approximativement $10^8$ dpm/µg.

Les dispositions relatives des fragments subgénomiques contenus dans les sondes vis-à-vis du génome entier de HIV-1 sont schématisées à la figure 3. Les différentes taches correspondent respectivement :

    - tache A : virus obtenus à partir d'une culture de cellules CEM C1.13 infectées par HIV-1,

    - tache B : virus obtenus à partir de cellules HUT-78 infectées par STLV-III,

    - taches C et D : isolats respectivement obtenus à partir des virus des deux patients africains susmentionnés,

    - tache E : extrait cellulaire témoin négatif obtenu à partir de cellules HUT-78 non infectées,

    - tache F : virus obtenu à partir d'un patient du Zaïre affecté par le SIDA et ayant été cultivé sur des lymphocytes T normaux en présence de TCGF.

Toutes les taches ont été obtenues avec une quantité de virus correspondant à 25.000 dpm en activité transcriptase inverse, sauf pour les taches C : 15.000 dpm.

Les observations faites ont été les suivantes :

Les ARNs génomiques des deux isolats de HIV-2, obtenus à partir de particules virales purifiées, n'ont hybridé avec aucune des sondes dans les conditions stringentes sus-indiquées, bien que les particules virales aient été isolées et purifiées à partir de surnageants de cultures de cellules hautement infectées témoignant d'une activité transcriptase inverse élevée.

Dans les conditions non stringentes sus-indiquées, les observations suivantes ont été faites.

Toutes les sondes ont hybridé de façon intense avec les ARNs génomiques obtenus des préparations témoins de HIV-1 et d'un autre isolat obtenu à partir d'un patient du Zaïre souffrant du SIDA.

Deux des sondes obtenues (nucléotides 990-1070 et 990-1260, toutes deux issues de la région gag de HIV-1) ont hybridé légèrement avec les taches des extraits des rétrovirus HIV-2; l'une seulement de ces deux sondes (nucléotides 990-1260) a aussi montré une légère hybridation avec la tache de STLV-III (figure 3). En ce qui concerne la sonde contenant un fragment de la région pol (nucléotides 2170-2240), on a observé une hybridation avec STLV-III et, mais de façon beaucoup plus faible, avec l'ARN de HIV-2. L'autre sonde de la région pol (nucléotides 3370-3640) n'a donné d'hybridation avec aucune des taches de HIV-2 et de STLV-III.

Enfin, la sonde modifiée par translation de coupure et contenant la totalité du gène env et LTR (nucléotides 5290-9130) de HIV-1 n'a hybridé ni avec les ARNs de STLV-III, ni avec ceux de HIV-2.

On remarquera encore qu'une autre sonde, qui contenait l'extrémité 5' du cadre de lecture de pol de HIV-1 (correspondant à la région protéase) n'a hybridé ni avec les ARNs de HIV-2, ni avec les ARNs de STLV-III.

Il résulte donc encore de ce qui précède que le virus HIV-2 paraît plus éloigné, sur le plan structural, du virus HIV-1 qu'il ne l'est de STLV-III. HIV-2 diffère néanmoins de façon significative de STLV-III, ce qui vient à l'appui des résultats différents observés quant aux capacités infectieuses des virus HIV-2 pratiquement nulles chez les singes, comparés aux capacités infectieuses certaines des STLV-III chez les mêmes espèces de singes.

Les cartes de restriction et les séquences de l'ARN génomique de HIV-2 ou des cADNs obtenus à partir de ces ARNs génomiques sont accessible à l'homme du métier, dès lors que les souches de HIV-2 déposées à la C.N.C.M. peuvent, après multiplication appropriée, mettre en ses mains le matériel génétique nécessaire aux déterminations de ces cartes de restriction et séquences nucléotidiques. Les conditions dans lesquelles la carte de restriction du génome de l'un des isolats de HIV-2 de cette invention ont été établies et les conditions dans lesquelles certaines des parties de ADNc dérivées de ces génomes ont été séquencées sont décrites ci-après.

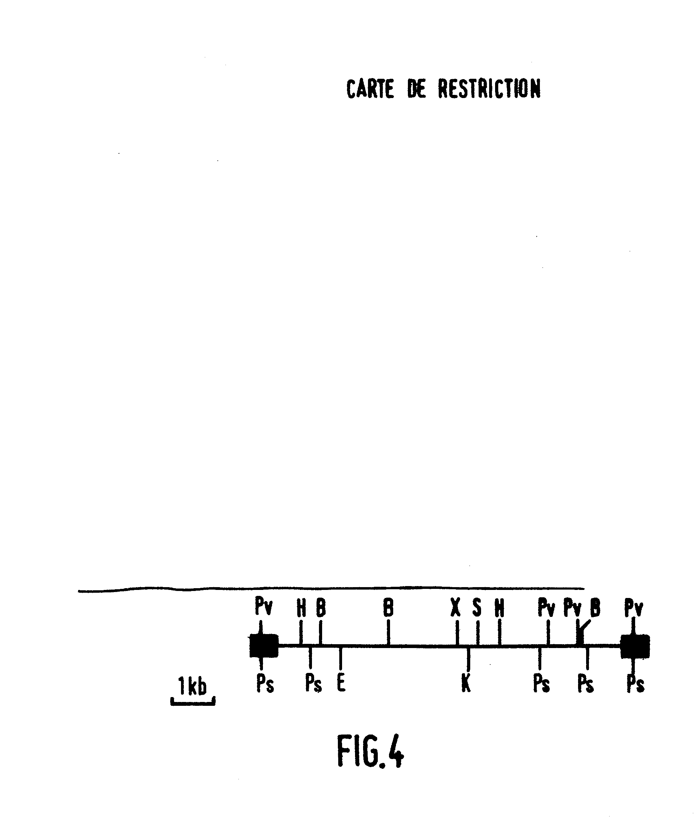

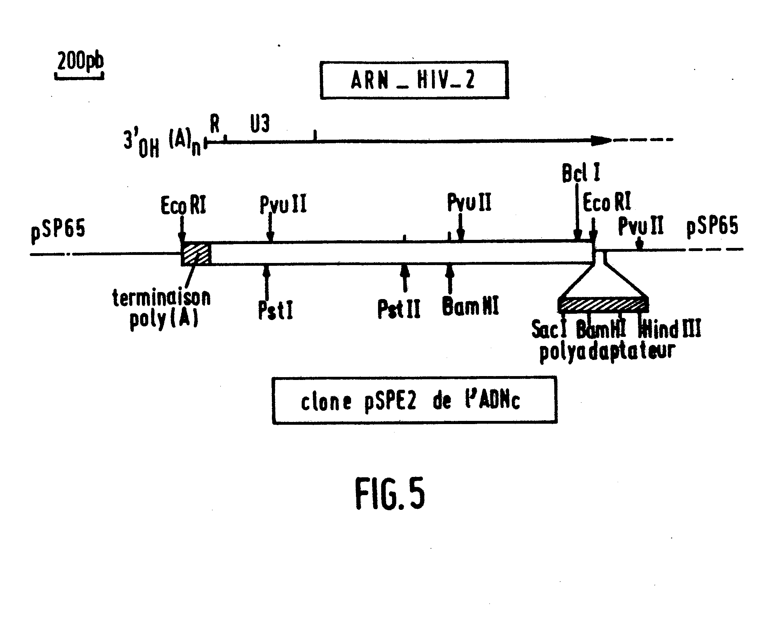

La carte de restriction du génome d'un rétrovirus représentatif des rétrovirus HIv-2 est représentée dans la figure 4. La carte de restriction d'un fragment important de cet ADNc apparaît à la figure 5. Enfin une partie de ce dernier fragment a été séquencé.

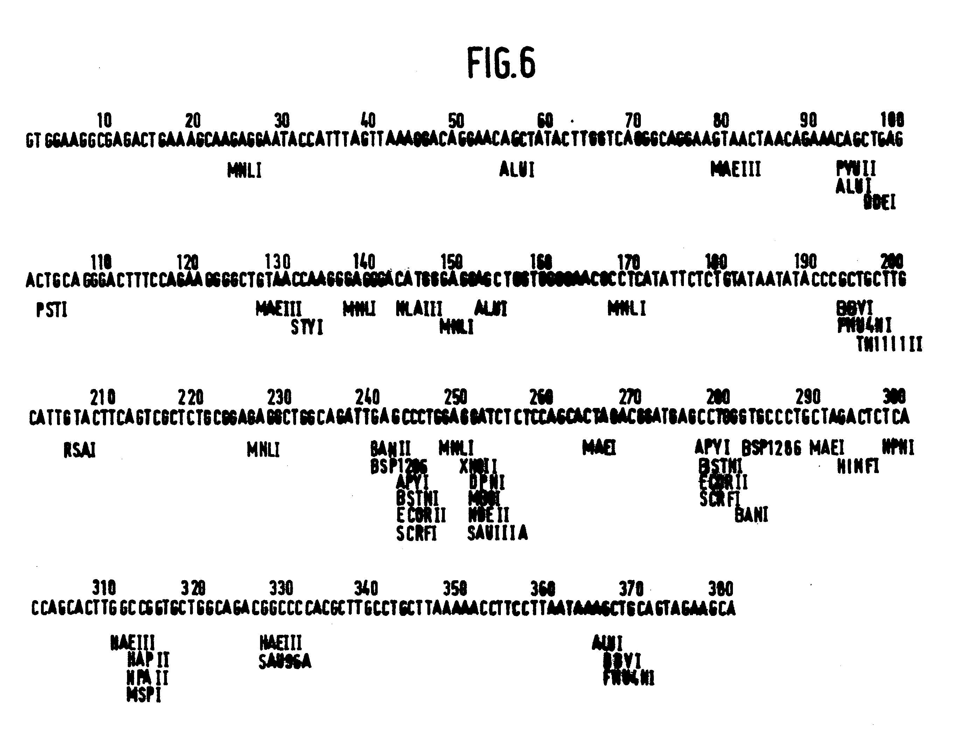

Cette séquence et un certain nombre des sites de restriction qu'elle contient apparaissent à la figure 6. L'ADNc entier cloné -ou des fragments clonés de cet ADNc-peuvent eux-mêmes être utilisés comme sondes d'hybridation spécifiques.

2) ADNc et fragments de cet ADNc, respectivement dérivés de l'ARN de HIV-2

Les conditions dans lesquelles l'ADNc susdit a été obtenu, sont décrites ci-après.

La première étape de fabrication de cet ADNc a compris la production d'un oligo (dT) servant d'amorce ou d'un brin d'ADNc initiateur, par la réalisation d'une réaction endogène activée par un détergent, utilisant la transcriptase inverse de HIV-2, sur des virions purifiés, obtenus à partir de surnageants de cellules CEM infectées. La lignée cellulaire CEM était une lignée cellulaire lymphoblastoïde CD4+ décrite par G.E. Foley et al. dans Cancer 18: 522-529 (1965), dont le contenu est considéré comme faisant partie de la présente description. Ces cellules CEM utilisées sont infectées avec un isolat ROD, qui s'est révélé produire de façon continue des quantités importantes de HIV-2.

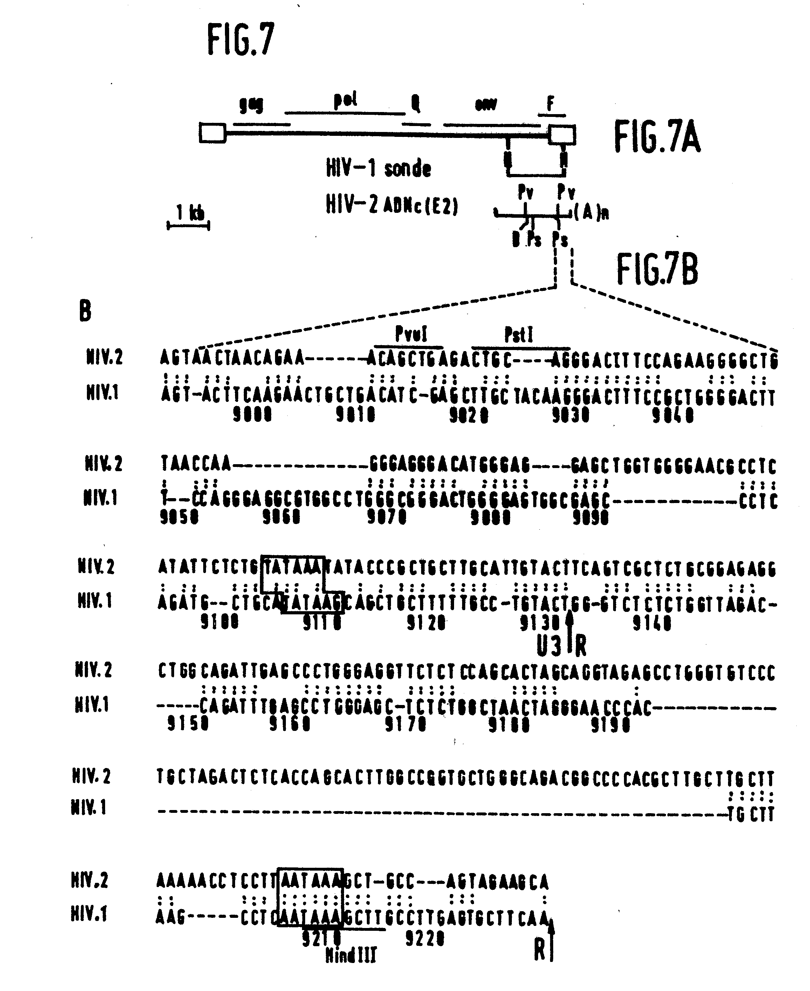

Après la synthèse du second brin (en présence de nucléotides et d'une ADN polymérase bactérienne), les cADNs double-brins ont été insérés dans un vecteur de phage bactérien M13 TG130. Une banque de phages de 10⁴ phages recombinants M13 a été obtenue et soumise à un tri in situ avec une sonde HIV-1. Celle-ci contient un fragment de 1.5kb issu de l'extrémité 3' de l'ADNc dérivée de l'ARN de l'isolat LAV (montré dans la figure 7 A). Environ 50 plaques positives ont été détectées, purifiées et caractérisées par une hybridation croisée des insérats et séquençage des extrémités.

Cette procédure a permis l'isolement de différents clones contenant des séquences approximativement complémentaires de l'extrémité 3' de l'ARN polyadénylé de la région de "répétition longue terminale" LTR (abréviation anglaise de "long terminal repeat" de HIV-1, décrite par S. Wain Hobson et al. dans Cell 40: 9-17 (1985)) dont le contenu est considéré ici comme faisant partie de la description.

Le plus important des insérats du groupe concerné de clones de M13 hybridant avec la région LTR3' de HIV-1, est un clone désigné par E2, d'environ 2 kb. Comme la région LTR3' de HIV-1, le clone E2 contient un signal AATAAA situé à environ 20 nucléotides en amont d'une partie terminale poly A, et une région LTR3' correspondant à celle de HIV-2. Après séquençage partiel, cette région LTR3' de HIV-2 s'est révélée présenter une parenté éloignée avec le domaine homologue de HIV-1.

La figure 5 est une carte de restriction du fragment de E2 (zone rectangulaire allongée) incorporé dans le plasmide pSPE2 qui le contient. Il comprend une partie de la région R, la région U3 de HIV-2. Le dessin ne fait pas apparaïtre les limites des régions R et U3.

La séquence d'une partie de E2 apparaît à la figure 6. Les positions de sites de restriction spécifiques y sont indiquées. Le faible degré de parenté entre les régions LTR3' de HIV-1 et HIV-2 est illustré par la figure 7. En effet, à peu près 50% seulement des nucléotides des deux LTR peuvent être mis en alignement (homologue de séquence d'environ 50%) au prix d'une centaine d'insertions ou de délétions. En comparaison, l'homologie de séquence des régions correspondantes des isolats américains et africains différents variants de HIV-1 est supérieure à 95%, sans insertion ni délétion.

Le clone E2 a été utilisé comme sonde spécifique d'HIV-2, pour l'identification sur filtre d'hybridation des séquences issues de HIV-2, contenues dans d'autres clones.

Cette sonde détecte également l'ARN génomique de HIV-2 dans des conditions stringentes. Elle permet, de même, la détection par la méthode dite de "Southern blot" sur l'ADN de cellules CEM ou analogues infectées avec un isolat ROD ou d'autres isolats de HIV-2. Aucun signal n'est détecté dans les mêmes conditions de stringence dans des essais d'hybridation de cette sonde avec des ADNc issus de cellules non infectées ou de cellules infectées par HIV-1. Ces résultats ont confirmé la nature exogène de HIV-2 vis-à-vis de HIV-1. Une espèce d'environ 10 kb, correspondant probablement à l'ADN viral non intégré, a été détecté à titre principal dans l'ADN non digéré de cellules infectées par HIV-2. Un autre ADN ayant une taille apparente de 6 kb correspondant peut être à une circulaire de l'ADN viral, a été détecté aussi.

Les autres parties du génome de HIV-2 ont été identifiées également. A cet effet, une banque génomique a été construite dans le phage lambda L47. Le phage lambda L47.1 a été décrit par W.A.M. Loenen et al. dans Gène 10: 249-259 (1980), publication dont le contenu est considéré comme faisant partie de la présent description.

La banque génomique est construite avec des fragments obtenus par digestion de l'ADN provenant de la lignée cellulaire CEM infectée avec HIV-2ROD, après digestion avec l'enzyme Sau3AI.

Environ 2 x 10⁶ plaques de recombinants ont été triées in situ avec un clone contenant l'insérat E2 marqué de l'ADNc de HIV-2. Dix phages recombinants ont été détectés sur plaques et purifiés. Les cartes de restriction de trois de ces phages, caractérisés par leur capacité d'hybridation en "Southern blot" avec l'insérat E2 dans des conditions stringentes, ainsi qu'avec des sondes subgénomiques de HIV-1 dans des conditions non-stringentes.

Un clone portant un insérat de 9.5 kb et dérivant de l'ADN viral circulaire entier, contenant le génome complet de HIV-2, a été identifié. Il a été désigné "Lambda ROD 4". Les deux autres clones, Lambda ROD 27 et Lambda ROD 35 dérivant de provirus intégrés, portant des séquences LTR des séquences codantes virales et des séquences adjacentes d'ADN cellulaire. Les différentes séquences découlent de la figure 8.

Des fragments des clones Lambda ont été sous-clonés dans plasmide vecteur plasmidique pUC 18. Les fragments issus de λ ROD 4, λ ROD 27 et λ ROD 35 et respectivement sous-clonés dans le susdit vecteur plasmide apparaissant également à la figure 8. Les sous-clones suivants ont été obtenus.

- pROD 27-5 dérive de Lambda ROD 27, contient une région de 5,2 kb du génome de HIV-II et des séquences cellulaires adjacentes (5' LTR et séquence virale condante 5' autour d'un site ECO RI).
- pROD 4.8 dérivé de Lambda ROD 4, contient un fragment Hind III d' environ 5 kb. Ce fragment correspond à la partie centrale du génome HIV-2.
- pROD 27-5' et pROD 4.8 contiennent des insérats de HIV-2 qui se chevauchent mutuellement.
- pROD 4.7 contient un fragment HIND III de 1.8 kb de Lambda ROD 4 ; ce fragment est placé dans la direction 3 vis-à-vis du fragment sous-cloné dans p ROD 4.8 et contient environ 0.8 kb de séquences virales codantes et une partie située entre les sites de clonage Bam H1 et Hind III du bras gauche du phage Lambda (Lambda L 47.1).
- pROD 35 contient toutes les séquences HIV-2 codantes dans la direction 3, vis-à-vis du site Eco R1, l'extrémité 3' LTR et environ 4 kb de séquences nucléiques adjacentes d'origine cellulaire.
- pROD 27-5' et pROD 35 contenus dans E.Coli HB 101, ont été déposés à la CNCM sous les numéros respectifs I-626 et I-633, le 21 novembre 1986.
- pROD 4.7 et pROD 4.8, contenus dans E.Coli TG1, ont été déposés à la CNCM respectivement sous les numéros 1-627 et 1-628, le 21 novembre 1986.

Le génome complet de HIV-2 ROD, dont la carte de restriction apparaît à la figure 4, a été reconstitué à partir de pROD 35, linéarisé au préalable avec Eco R1, de pROD 27-5'. L'insérat Eco R1 de pROD 27-5 a été ligaturé dans l'orientation correcte dans le site EcoRI de pROD 35.

Le degré de parenté entre HIV-2 et les autres rétrovirus humains ou simiens a été apprécié par des expériences d'hybridation mutuelle. L'homologie relative entre les différentes régions de génomes de HIV-1 et de HIV-2 a été déterminée par des essais d'hybridation de fragments respectivement issus de génome de HIV-1 cloné et de Lambda ROD 4 marqué radioactivement. Les positions relatives de ces fragments (numérotés de 1 à 11) vis à vis du génome de HIV-1 apparaîssant au bas de la figure 9.

Même dans des conditions de très faible stringence (Tm-42°C.) les génomes de HIV-1 et HIV-2 n'hybrident qu'aux niveaux de leurs gènes respectifs gag (taches 1 et 2), des régions reverse transcriptase dans pol (tache 3), des régions d'extrémités de pol, des gènes O (ou sor) (tache 5) et des gènes F (ou 3' orf) et 3' LTR (tache 11). Le fragment HIV-1 utilisé pour détecter les premiers clones ADNc de HIV-2 correspond au sous-clone de la tache 11, qui hybride relativement bien avec HIV-2 dans des conditions non stringentes. Un signal provenant de la tache 5 est le seul persistant après un lavage stringent. Le gène d'enveloppe, la région du gène tat et une partie de pol apparaissent très divergents. Ces données, ainsi que la séquence obtenue avec LTR (figure 3) démontrent que HIV-2 n'est pas (en tous les cas au niveau de son enveloppe) un variant de HIV-1.

On observe que HIV-2 est plus proche de SIV (décrit par M.D.Daniel et al. dans Science 228: 1201-1204 (1985), dont le contenu doit être considéré comme faisant partie de la présente description) qu'il ne l'est de HIV-1.

Toutes les protéines de SIV, y compris la protéine de l'enveloppe, sont immuno-précipitées par des sérums de patients infectés par HIV-2, tandis que la réactivité sérologique croisée de HIV-1 et HIV-2 est limitée aux protéines du noyau. Cependant SIV et HIV-2 peuvent être distingués par les différences mentionnées plus haut au niveau des poids moléculaires de leurs protéines.

En ce qui concerne les séquences nucléotidiques, on remarque aussi que HIV-2 a une parenté avec SIV.

De plus, la caractérisation de HIV-2 permet aussi de délimiter le domaine de la glycoprotéine d'enveloppe qui est responsable de la fixation du virus à la surface des cellucles cibles et de l'internalisation ultérieure du virus. L'interaction se fait par l'intermédiaire de la molécule CD4 elle-même et ils emble que HIV-1 et HIV-2 utilisent le même récepteur. Aussi, bien qu'il existe de grandes différences entre les gènes env de HIV-1 et HIV-2, les domaines homologues restreints des enveloppes des deux HIV peuvent-ils être considérés comme constituants de fixation à un récepteur commun des lymphocytes T4. Ces sites sont appelés à constituer des épitopes porteurs de l'immunogénicité de peptides qui pourraient être utilisés pour susciter chez l'homme une réponse immunitaire protectrice contre les virus HIV.

Des séquences avantageuses pour la constitution de sondes dans des réactions d'hybridation avec le matériel génétique de patients porteurs de virus ou de provirus, notamment pour détecter la présence d'ARN de virus HIV-é dans leurs lymphocytes contiennent une séquence nucléotidique résultant de combinaison des fragments de 5 kb de HindIII de ROD 4 et de l'ADNc E2. Les essais peuvent être réalisés selon toutes méthodes notamment selon les techniques de "Northern blot", "Southern blot" et "dot blot".

Des caractéristiques supplémentaires de l'invention apparaîtront encore de façon non limitative au cours de la description qui suit d'exemples d'identification de certaines parties du génome rétroviral et de la production d'un certain nombre d'ADNs recombinants mettant en jeu diverses parties d'un ADNc dérivé du génome rétroviral de HIV-2.

EXEMPLE I

Sonde ADN, pour l'utilisation dans les kits de diagnostic de HIV-2.

Un ADNc complémentaire de l'ARN du génome obtenu à partir de virions purifiés, a été préparé selon la méthode suivante :

Le surnageant obtenu après 48 heures de culture de cellules CEM infectées par un isolat HIV-2 ROD de HIV-2 a été ultracentrifugé. Le culot de centrifugation contenant le virion a été centrigué sur gradient de sucrose pour former un nouveau culot de centrifugation substantiellement par la même méthode que celle décrite dans la demande de brevet européen 84/401 234/0.138.667 déjà mentionnée.

La préparation de HIV-2 purifié a été utilisée pour la synthèse d'ADNc en mettant en oeuvre une réaction endogène, activée par un détergent.

En résumé, la préparation du virion a été ajoutée à un mélange de réaction contenant 50mM TrisHCl, 5mM MgCl2, 10mM DTT, 0.025% du détergent commercialisé sous la marque TRITON, et 50 µM de chacun des 4 déoxynucleoside-triphosphates et un initiateur oligo (dT). La réaction a été conduite pendant 90 minutes à 37°C.

Après l'extraction au phénol des protéines présentes dans le premier milieu de réaction, la seconde chaîne de l'ADNc a été synthétisée en présence de RNAse, ADN polymérase 1 de E.coli et des 4 déoxynucléotides, pendant 1 heure à 15°C et 1 heure à 22°C. Des extrémités franches ont été créées sur cette double chaîne ADNc, par l'action de la T4 ADN polymérase. Tous les réactifs de ce procédé sont disponibles dans le commerce (kit ADNc de la Société AMERSHAM) et ont été utilisés comme recommandé par le fournisseur.

Après (1) ligation d'adaptateurs (linkers) contenant un site EcoR1 (commercialisés par Pharmacia) aux extrémités franches de l'ADNc en présence d'une ligase ADN T4 (commercialisée par BIOLABS) (2), digestion de ces "linkers" par l'endonucléase de restriction EcoR1, et (3) élimination des fragments de "linker" par une filtration sur gel (colonne du gel commercialisé sous la marque ULTROGEL) AcA 34 (LKB-IBF), l'ADNc est inséré dans un vecteur M 13 TG 130 clivé par EcoR1. Une banque d'ADMcs a été obtenue après transformation de la souche de E.coli TG1. On a obtenu approximativement $10^4$ plaques de recombinants M13.

Pour sélectionner dans la banque d'ADNcs des clones M13 recombinants contenant l'ADNc de HIV-2, la technique d'hybridation sur plaque a été utilisée. L'ADN des plaques M13 a été transféré sur des filtres de nitrocellulose, et hybridé à des sondes de HIV-1 subgénomiques dérivées du clone "lambda J19" d'un virus LAV (ou HIV) décrit dans la demande de brevet européen. Cette sonde comprenait un insérat constitué par une partie ayant une longueur approximative de 1500 paires de bases (pb) de l'ADN de HIV-1. Cet insérat était délimité par deux sites de restriction HindIII respectivement à l'intérieur du cadre de lecture ouverte du gène "env" et dans le segment R de l'extrémité 3' LTR de HIV-1. Cette sonde contenait l'extrémité 3' du gène env, la totalité du gène F, le segment U3 et une partie du segment R de LTR, d'une longueur approximative de 1500 paires de bases (pb).

La sonde contenant l'insérat HindIII de 1,5kb a été marquée avec du $^{32}$P dCTP et dTTP (3000 Ci x 10 $^{-3}$ mole) par incubation de la sonde en présence d'initiateurs et d'ADN polymerase I de Klenow, pendant 4 heures à 15°C (en utilisant un kit de AMERSHAM). Les essais d'hybridation des clones d'ADNc de la banque ont été conduits pendant toute la nuit dans des conditions de faible stringence, au sein d'une solution d'un milieu d'hybridation contenant 5X SSC, 5X Denhart, 25% de formamide, 100 µg/ml d'ADN de sperme de saumon dénaturé et la sonde marquée (2 x $10^7$ cpm avec une spécificité de $10^9$ cpm/ug) à 37°C. Les filtres ont été soumis à trois étapes de lavage, successivement en présence des trois solutions dont les compositions sont indiquées ci-après: Lavage no 1:5XSSC, 0.1% SDS,à 25°C pendant 4x15 minutes Lavage no 2:2XSSC, 0.1% SDS,à 42°C pendant 2x30 minutes Lavage no 3:0.1XSSC,0.1% SDS, à 65°C pendant 2x30minutes Chaque lavage est suivi par une autoradiographie des filtres.

Plusieurs clones positifs ont été détectés après le lavage no 1 et l'ont encore été après le lavage no 2. Cependant, tous les signaux disparaissaient après le lavage no 3. Ceci indique que les clones positifs n'avaient avec le génome de HIV-1 qu'une faible parenté, néanmoins suffisante pour effectuer la susdite sélection. Les clones positifs ont été repiqués, redéposés sur des plaques et de nouveau hybridés avec la même sonde dans les conditions de stringence correspondant au lavage no 1. La majorité d'entre-eux étaient encore positifs.

Les clones ont aussi été sélectionnés en mettant en oeuvre une sonde d'ADN humain total dans des conditions de stringence moyenne et par hybridation dans 5X SSC, 5X Denhart, et 40% de formamide, suivie d'un lavage dans 1X SSC, 0.1% SDS, à 50°C. Aucun des clones préalablement positifs n'a été détecté, par conséquent ne correspondait pas à l'ADN répétitif spécifique ou à l'ADNc de l'ARN ribosomal.

Les clones recombinants M13 positifs ont été cultivés dans un milieu liquide et caractérisés :

(1) Taille de leur insertion :

Un ADN type simple brin de M13 a été obtenu à partir de chaque clone individuel, et la synthèse du second brin a été conduite avec une séquence initiatrice 17-mer M13 et l'enzyme de Klenow. Les insérats ont été excisés à l'aide d'EcoR1 (BOEHRINGER) et analysés par électrophorèse sur gel d'agarose. La plupart des inserats contenaient de 200 à 600 et 200 pb, à l'exception du clone dénommé E2.1, qui avait approximativement une longueur de 2 Kpb.

(2) Analyse de la séquence nucléotidique :

Plusieurs clones ont été partiellement séquencés, en utilisant la méthode dideoxy de Sanger et al. décrite dans Proc. Natl. Acad. Sci. 74:5463-7 (1977), faisant partie de la présente description. Différents clones indépendants contenaient des séquences nucléotidiques similaires, à l'exception des chaînes poly-A à leurs extrémités 3' dont les longueurs étaient différentes. Ces résultats démontrent que ces clones ADNc étaient dérivés de la matrice d'ARN. L'analyse de séquence détaillée de ces clones ADNc, comprenant l'extrémité 3' du génome de HIV-2, a montré une parenté limitée avec HIV-1.

(3) Hybridation avec l'ARN et l'ADN génomique de HIV-2.

(a) Obtention de l'ARN génomique de HIV-2 : Un surnageant infecté a été centrifugé (50.000 rotations, 30 minutes). Le culot du dépôt a été resuspendu dans 10mM Tris pH 7.5 1mM EDTA, 0.1% SDS. L'un des clones d'insertion F1.1 a été marqué et utilisé comme sonde pour l'hybridation avec l'ARN génomique de différents isolats viraux, conformément à la technique de "dot-blot".

La technique "dot-blot" comprenait les étapes suivantes :

(i) déposer l'échantillon (lysat HIV-2) en taches sur une membrane de nitrocellulose préalablement trempée dans 20X SSC (3M NaCl, 0.3M de citrate de sodium) et séchée à l'air, (ii) faire cuire la membrane pendant 2 heures à 80°C., et (iii) effectuer l'hybridation.

Cette hybridation a été conduite dans des conditions de haute stringence (5X SSC, 5X Denhart, 50% formamide à 42°C). Elle a été suivie d'un lavage dans 0.1X SSC, 0.1% SDS à 65°C. Dans ces conditions, la sonde hybride fortement aux tâches provenant de deux isolats indépendants de HIV-2, incluant le LAV-II ROD, dont l'ADNc cloné était issu. Un faible signal d'hybridation a été détecté avec la tache formée par STLV-IIImac (Virus T-Lymphotropique de Singe (également connu comme "SIV") de type III, macaque), et aucune hybridation n'a été détectée avec les isolats de HIV-1.

Les expériences de "Southern Blot", mettant en jeu le clone E-2.1 contenant l'inserat de 2Kb en tant que sonde marquée au $^{32}$P, ne manifestèrent aucune hybridation avec des ADNs de cellules non infectées, mais détectaient des bandes dans des cellules détachées infectées par HIV-2 dans des conditions de forte stringence. HIV-2 montre un polymorphisme à des niveaux de sa carte de restriction équivalents à ceux des cartes de restriction de HIV-1. Avec de l'ADN cellulaire complet de cellules infectées, deux sortes de signaux sont détectés par la méthode de "Southern Blot" : (1) dans des fractions d'ADN ayant des poids moléculaires PM d'environ 20kb et plus dans le cas de formes intégrées du virus, et (2) dans les fractions de PM plus faibles (9,10kb), dans le cas de virus non intégré au génome.

Ces caractéristiques sont hautement spécifiques d'un rétrovirus.

Certaines expériences effectuées avec STLV-III (SIV-3) de cellules infectées ont permis de constater que le rétrovirus simien est relativement distant de HIV-2 (le signal est uniquement détecté dans des conditions de faible stringence). Ces expériences montrent que les susdites sondes permettent la détection spécifique de HIV-2.

(4) Sous-clonage de l'ADNc de HIV-2 dans un vecteur plasmide bactérien :

Le clone M13 positif, E 2-1 a été sélectionné et sous-cloné dans un vecteur plasmide. L'ADN du phage M13 (TG 130) recombinant E-2 a été purifié sous forme d'un ADN simple brin (M-13-ROD-E2) contenant l'inserat de 2 kb, contenant la portion 3' du génome de HIV-2 (obtenu à partir de HIV-2 ROD). Cet inserat a été transféré dans le plasmide pSP65, décrit par Melton, D.A. dans 357 Nucleic Acid Res. 12:035-7056 (1984).

Une seconde chaîne a été construite in vitro en présence de la séquence d'initiateur 17 mer (AMERSHAM), des quatre nucléotides A,C,T,G, et de l'ADN polymérase I (Klenow). L'inserat EcoR1 a été excisé par digestion EcoR1 et purifiée sur gel d'agarose, puis ligué à pSP65, lui-même préalablement digéré avec EcoR1. Le mélange de ligation a été utilisé pour transformer la souche E.coli DH1 et des recombinants ont été sélectionnés grâce à leur capacité de résistance à l'ampicilline. Les recombinants identifiés ont été cultivés sur milieu LB (Luria medium) contenant 50 µg/ml d'ampicilline. Ces plasmides recombinants ont été purifiés et contrôlés quant à la présence du fragment inséré correct.

L'un des clones obtenus désigné par la référence pSPE2, a été déposé à la CNCM à PARIS, France, sous le no d'accès I-595 le 5 Septembre 1986.

Les insérats dérivés des ADNc de HIV-2 et qui se trouvaient insérés dans la sonde susdite contenaient la séquence de nucléotides qui a été définie plus haut, en rapport avec une partie de E2.

EXEMPLE II

Clonage d'un ADNc complémentaire à l'ADN complémentaire à l'ARN génomique de virions de HIV-2

Des virions HIV-2 ont été purifiés à partir de 5 litres d'un surnageant d'une culture d'une lignée CEM infectée avec un isolat ROD. Un premier brin d'ADNc a été synthétisé au contact de virus purifié sédimenté, en présence d'un initiateur oligo (dt) et en mettant en oeuvre une réaction endogène activée par un détergent, selon la technique décrite par Alizon et al, Nature 312 : 757-760 (1984). Les hybrides ARN-ADNc ont été purifiés par une extraction par un mélange phénolchloroforme et par une précipitation à l'éthanol. Le second brin d'ADNc a été produit en présence d'ADN polymérase-I/RNAse H selon la méthode décrite par Gubler et Hoffman. La description de cet article est considérée comme faisant partie de la présente description.

L'ADNc double brin a été muni d'extrémités franches en présence d'ADN polymérase T4 en utilisant les constituants d'un kit de synthèse d'ADNc commercialisé par AMERSHAM.

Des adaptateurs (linker) Eco R1 commercialisés par PHARMACIA, ont été fixés à l'extrémité de l'ADNc; l'ADNc ainsi modifié a été inséré après digestion en présence de Eco R1 dans un vecteur de phage déphosphorilé M13tg130 lui-même digéré par Eco R1, également commercialisé par AMERSHAM. Une bande d'ADNc a été obtenu après transformation de la souche E.coli TG1. Des plaques de recombinants (10[4]) ont été triés in situ sur des filtres permettant des répliques par hybridation avec le clone J19 contenant le fragment Hind III de 1,5kb déjà mentioné plus haut, issu de HIV-1.

Les filtres ont été préhybridés en présence d'un milieu contenant 5 x SSC, 5 x solution DENHARDT, formamide à 25%, de l'ADN dénaturé de sperme de saumon (100 microgrammes/mml) à 37°C, pendant 4 heures, puis hybridé pendant 16 heures dans le même tampon (Tm -42°C) en présence d'un supplément de sonde marquée (4 x 10[7]cbm), pour fournir une solution finale de tampon d'hybridation contenant 10[6]cpm/ml). Le lavage a été fait avec une solution 5 x SSC, 0,1% SDS à 25°C pendant 2 heures (étant entendu que 20 x SSC correspond à une solution NaCl 3M et de citrate de sodium 0,3M. Les plaques répondant positivement ont été purifiées et les ADNs mono-brins de M13 ont été préparés et leurs extrémités ont été séquencées selon la méthode de Sanger et al.

Hybridation d'un ADN de cellules infectées par HIV-1 et HIV-2 et d'ARNs de HIV-1, HIV-2 et de virions SIV respectivement avec une sonde dérivée d'un ADNc cloné de HIV-2.

Les ADNs ont été extraits de cellules CEM infectées produisant de façon continue respectivement HIV-1 et HIV-2. Des échantillons d'ADN de ces deux rétrovirus, digérés pour certains par 20 ug de PstI, non digérés pour d'autres ont été soumis à une electrophorèse sur gel d'agarose à 0,8% et transféré par la méthode "Southern" sur membrane de nylon. Des petits volumes de surnageant infecté, repris dans un tampon NTE contenant 0,1% de SDS ayant les mêmes activités de transcriptase inverse ont été déposés sur nitrocellulose, qui avait été trempé auparavant dans une solution 2 x SSC.

Une préhybridation a été faite dans une solution contenant 50% de formamide, 5 x SSC, 5 x Denhardt, et 100 mg/ml d'ADN de sperme de saumon dénaturé pendant 4 heures à 43°C. Une hybridation a été conduite dans le même tampon, auquel avait été ajouté 10% de sulphate de dextran, et $10^6$ cpm/ml. d'insérat marqué E2 (activité spécifique $10^9$ cpm/ug) pendant 16 heures à 42°C. Deux lavages ont ensuite été faits avec une solution 0.1 x SSC, 0.1% SDS pendant 30 mn chacun. Après l'exposition pendant 16 heures à un écran d'intensification la tache Southern est déshybridée dans 0.4 N NaOH, neutralisée et réhybridée dans les mêmes conditions à la sonde HIV-1 marquée avec $10^9$ cpm/ug.

## EXEMPLE III

Clonage dans le Phage Lamdba de l'ADN complet du provirus de HIV-2.

L'ADN des cellules CEM infectées par HIV-IIROD (figure 2, bandes a et c) est partiellement digéré avec Sau3AI. La fraction 9-15 kb a été sélectionnée sur gradient de sucrose a 5-40% et liguée aux bras BamHI du vecteur lambda L47.1. Les plaques (2 x $10^6$) obtenues après empaquetage in vitro et dépôt sur souche E.coli LA 101 ont été triées in situ par hybridation avec l'insérat de l'ADNc cloné E2. Approximativement, 10 clones positifs ont été purifiés sur plaque et propagés sur E.coli C600 recBC. Les clones lamdba ROD 4, ROD 27 et ROD 35 ont été amplifiés et leurs ADN caractérisés par établissement de leurs cartes de restriction et par hybridation par la méthode de "Southern" avec le clone ADNc de HIV-II dans des conditions stringentes et avec les sondes gag-pol de HIV-1 dans des conditions non stringentes.

La figure 8 présente de façon schématique les structures de 3 des phages recombinants obtenus ROD 4, ROD 27 et ROD 35.

Les parties rectangulaires allongées de ces schémas correspondent à des séquences pro-virales issues des ADNs des cellules CEM initialement infectées, les parties blanches correspondant à des séquences rétrovirales, les parties grisées à des parties des ADNs cellulaires et les parties en noir au LTR dans lesdites fréquences virales.

Les traits fins désignés par les lettres L et R correspondent aux bras issus du vecteur de phage Lambda L47.1 ayant servi au clonage.

Certains des sites des restrictions ont également été indiqués : il s'agit plus particulièrement des sites suivants :
B : BamHI ; E ; EcoRI ; H: HindIII ; K: KpnI ; Ps: PstI;Pv : PvuII ; S: SacI ; X: XbaI.

Ces séquences virales ont des parties en commun avec la séquence E2. Les positions relatives de ces parties déterminées par hybridation avec E2, apparaissent également dans les figures.

ROD 4 est dérivé d'un ADN viral circulaire. ROD 27 et ROD 35 sont dérivés de provirus intégrés dans une structure ADN cellulaire.

Enfin, on voit apparaître dans ces figures les insérats sous-clonés dans les conditions qui ont été indiquées plus haut et leurs positions relatives vis-à-vis des séquences correspondantes de ROD 4, ROD 27 et ROD 35.

Il s'agit plus particulièrement des insérats des plasmides pROD 27-5', pROD 35-3', pROD 4.6, pROD 4.8 et pROD 4.7.

La figure 9 est une représentation des intensités relatives des taches d'hybridation qui ont été réalisées entre ROD-4 et des sous-fragments 1 à 11 respectivement issus des différentes parties d'un ADN à chaîne unique issu de sous-clone de M13 contenant un acide nucléique dérivé du génome entier de LAV. Les positions relatives de ces divers fragments vis-à-vis du génome entier de LAV (déterminé par séquençage) sont indiquées dans la partie inférieure de la figure. Le point 12 correspond à une tache témoin réalisé en mettant en oeuvre un ADN témoin du phage Lambda.

Les essais d'hybridation dans la méthode de transfert par tache (dot-blot) ont été réalisés dans les conditions de faible stringence de l'exemple II. En utilisant à titre de sonde, le recombinant Lambda ROD 4 contenant l'ADNc total de HIV 2. Les lavages ont ensuite été faits successivement dans les conditions suivantes :
2 x SSC, 0.1% SDS à 25°C. (Tm -42°C), 1 x SSC, 0.1 % SDS à 60°C. (Tm -20°C), et 0.1 x SSC, 0.1% SDS à 60°C.
(Tm -3°C).

Les taches représentées ont été obtenues après exposition radiographique pendant 1 nuit.

## EXEMPLE IV

Test de diagnostic in vitro de la présence du virus HIV-2 dans un milieu biologique.

MATERIEL ET METHODES

PATIENTS

Les patients infectés par HIV-2 ont été sélectionnés parmi des personnes admises à l'hôpital Egas Moniz de LISBONNE pour hospitalisation ou pour consultation, entre septembre 1985 et septembre 1986.

Pour réaliser cette sélection, tous les individus d'origine africaine ou ayant séjourné en Afrique ont subi un test sérologique à la fois pour rechercher la présence d'anticorps contre HIV-I (immuno-fluorescence-IFA- et/ou ELISA) et contre HIV-2 (IFA). Seuls les patients pour lesquels on a prouvé sérologiquement l'infection avec HIV-2 ont été retenus dans cette étude.

Isolation du virus.

Chez 12 patients, l'isolation de HIV a été conduite comme on le rappelle brièvement. Les lymphocytes périphériques sanguins des patients (PBL) ont été stimulés avec PHA, cultivés en coculture avec des PBLs normaux humains stimulés par PHA et maintenus en présence d'interleukine-2 (IL-2). Les cultures ont été contrôlées afin de repérer la présence éventuelle d'un effet cytopathogénique (CPE) et afin de mesurer l'activité de transcriptase inverse (RT) dans le surnageant.

Test d'immunofluorescence (IFA)

Des plaques IFA ont été préparées comme suit : des cellules MOLT-4 infectées avec HIV-2 ont été lavées 2 fois dans PBS et étalées sur des lames de verre IFA ($10^4$ cellules par puits), puis séchées à l'air et fixées par de l'acétone à froid. Pour le test IFA, ces cellules ont été soumises à une réaction, le sérum de test étant dilué à 1/10, pendant 45′ à 37°C, ont été lavées, séchées et soumises à une réaction avec de la fluorescéine conjuguée à des IgG, A, M anti-humaines de chèvre (dilué à 1/100) pendant 30 minutes à 37°C. Après le lavage, les cellules ont été colorées avec du bleu Evans, à 0,006%, montées dans un mélange de 90% de glycérol, 10% de PBS et examinées au microscope à fluorescence.

ELISA

Certains sérums de patients ont été examinés pour déterminer la présence d'anticorps anti-HIV-1 en utilisant les tests sérologiques disponibles dans le commerce sous la dénomination ELAVIA (Pasteur Diagnostics) ou ABBOTT.

Test de radio-immunoprécipitation (RIPA)

Des cellules CEM infectées avec HIV-1 ou HIV-2 ont été cultivées en présence de $^{35}S$ cystéine (200 microCi/ml) pendant 16 heures. Le surnageant a été recueilli, les particules virales ont été centrifugées et lysées sur un tampon RIPA (Tris-HC1 50mM pH 7.5, NaC1 150mM, EDTA 1mM, 1% Triton X100, Sodium deoxycholate 0.1%, SDS 0.1%). Pour chaque réaction, 50 microlitres de lysat dilué correspondant à $10^5$ cpm ont été mis en réaction avec 5 microlitres d'un sérum test pendant 18 heures à 4°C. Des complexes immuns ont été liés grâce à une protéine A fixée sur du Sépharose PHARMACIA lavés, et élués pendant une ébullition de 2 minutes. Les antigènes élués ont été analysés par électrophorèse sur gel SDS-polyacrylamide et par autographie.

Hybridation Dot-blot

Des virus isolés dans des PBLs de patients ont été centrifugés et lysés dans Tris-HCl 10mM pH 7.5, NaCl 10mM, EDTA 1mM, SDS 0.5%. Un microlitre de chaque lysat, correspondant à une activité RT de 50.000 cpm a été déposé sur un support de nitrocellulose. Une hybridation et un lavage ont été réalisés dans des conditions de forte stringence: hybridation dans 6X SSC, 5X Denhart, 50% de formamide, pendant 18 heures à 42°C ; et lavage dans 0.1X SSC, 0.1% SDS 65°C. On a utilisé des sondes HIV-1 et HIV-2, marquées au 32p (activité spécifique de $10^8$ cpm/microgramme). La sonde HIV-1 correspond au génome complet de l'isolat LAV$_{BRU}$, et la sonde HIV-2 dérive d'un ADN cloné de 2 kilobases de l'isolat LAV-2$_{ROD}$.

RESULTATS

Population des patients

30 patients chez lesquels on décelait une infection par HIV-2 par mise en évidence sérologique ou virale, ont été étudiés. Parmi ces 30 patients on comptait 12 hommes et 18 femmes. La moyenne d'âge était de 35 ans pour un intervalle de 11 à 55 ans. Tous les patients excepté 1, étaient restés plusieurs années en Afrique de l'Ouest : 26 étaient nés et vivaient en Guinée-Bissau et 2 étaient originaires des Iles du Cap-Vert. 1 patient était un garçon de 11 ans de l'Angola, qui avait vécu dans les Iles du Cap-Vert pendant plusieurs années. Le seul européen de la population étudiée était un Portugais de 40 ans qui avait vécu pendant 8 ans au Zaïre, mais démentait tout séjour en Afrique de l'Ouest.

Présentation clinique

Parmi les 30 patients, 17 avaient un SIDA, conformément aux critères reconnus CDC. Le symptôme majeur chez ces patients était la diarrhée chronique, accompagnée dans la plupart des cas d'une perte de poids de plus de 10 kg. en 1 an. Chez 10 patients la diarrhée était associée avec la présence d'une infection intestinale soudaine ; pour 7 cas, l'agent pathogène était le seul Isospora belli, 1 patient était atteint uniquement par Cryptosporidium et 2 avaient à la fois les 2 agents pathogènes. Dans 3 cas, aucun agent pathogène intestinal inattendu n'a été révélé. Parmi les 17 cas de malades atteints de SIDA, on a diagnostiqué chez 8 des candidoses oesophagiennes. 6 patients atteints du SIDA présentaient des symptômes respiratoires. Une tuberculose pulmonaire a été diagnostiquée chez 2 d'entre eux et une microbactérie différente non identifiée a été décelée chez l'un d'entre eux.

2 patients souffraient d'une aspergilose pulmonaire à la suite d'une tuberculose. 2 autres patients atteints du SIDA présentaient des phases récurrentes de pneumonie sans identification de l'agent pathogène, et 1 patient avait une pneumonie du type pneumocystis carinii, qui fut diagnostiquée uniquement post-mortem. Quatre des 17 patients atteints du SIDA avaient un sarcome de Kaposis: dans 3 cas il est apparu comme limité à la peau et chez un autre patient un examen post-mortem a révélé des lésions disséminées au niveau viscéral. Des désordres du système nerveux central sont apparus chez 2 patients atteints du SIDA : 1 avait un lymphome cérébral, et l'autre avait une encéphalite subaiguë de cause inconnue.

4 patients présentaient les symptômes du complexe lié au SIDA (ARC) :

2 avaient des lymphadénopathies diffuses avec fièvre persistante, 1 avait une diarrhée chronique avec perte de poids, et 1 avait des phases récurrentes de bronchopneumonie et des lymphadénopathies multiples.

Parmi les 9 patients restants, 6 n'avaient aucun symptôme rattachable à l'infection par HIV, 1 était atteint uniquement d'une tuberculose pulmonaire , 1 avait uniquement des lymphadénopathies diffuses persistantes et 1 présentait une syphillis neurologique. Pendant la période d'étude de 12 mois, 7 patients sont morts, tous présentant un SIDA.

Etude sérologique

Chaque patient fait l'objet d'au moins un test sérologique pour rechercher la présence d'anticorps contre à la fois HIV-1 et HIV-2. Tous les sérums des patients ont été testés par test IFA afin de repérer les anticorps contre HIV-2 et tous se sont révélés positifs. Parmi eux, 21 furent aussi testés pour les anticorps contre HIV-2 par un test RIPA : tous ont précipité de façon claire la glycoprotéine de haut poids moléculaire de l'enveloppe du virus, désignée par gp140, et 16 d'entre eux ont réagi également avec la protéine majeure du noyau p26, alors qu'une seulement réagit avec une autre protéine virale du noyau, désignée par p16.

Dans ces sérums on a recherché la présence d'anticorps présentant des réactions croisées avec HIV-I en utilisant les différents tests. Un test IFA a été utilisé pour 24 sérums : 12 sont négatifs, 10 sont faiblement réactifs, et 2 positifs. Dans un test ELISA, 21 sérums ont été testés : 16 étaient négatifs, et 5 étaient positifs. Finalement, on a recherché dans 11 sérums la présence d'anticorps contre les protéines de HIV-1 par la méthode RIPA.

3 d'entre eux n'ont absolument pas réagi, et ceci avec aucune protéine virale, 2 seulement ont précipité avec la protéine p34, issue du gène pol, et 5 ont réagi avec la protéine p25, protéine majeure du noyau. 2 sérums ont réagi seulement faiblement avec la glyco-protéine gp110 de l'enveloppe du virus HIV-1. Ces deux sérums ainsi que tous les sérums positifs lors des tests IFA ou ELISA concernant les anticorps anti HIV-1 ont réagi fortement avec la gp140 de HIV-2 dans un test RIPA, indiquant une infection avec HIV-2 plutôt qu'avec HIV-1. Seulement 1 patient, qui n'est pas inclu dans la population étudiée, a été reconnu sérologiquement comme étant infecté par HIV-1 et non par HIV-2. Ce patient était une femme de 21 ans de l'Afrique Centrale (Ile Sao Tome) présentant un SIDA.

Isolation du virus

L'isolation des virus dans les lymphocytes périphériques du sang a été pratiquée sur 12 patients. Un HIV a été isolé chez 11 d'entre deux, grâce à la présence d'un effet cytopathogénique, et à la mesure d'un pic des particules associées à l'activité de transcriptase inverse dans les surnageants de culture.

Ces 11 isolats ont été identifiés comme étant des isolats de HIV-2 en utilisant la technique d'hybridation "dot-blot". Les taches virales obtenues à partir de 10 isolats ont montré une hybridation forte dans les conditions stringentes d'hybridation et de lavage avec une sonde HIV-2 dérivée d'un ADNc cloné de HIV-2, alors qu'aucune d'entre-elles n'hybridait avec les sondes HIV-1 dans les mêmes conditions. Un isolat hybridait seulement faiblement avec une sonde HIV-2, mais n'hybridait pas avec une sonde HIV-1.

Evaluation immunologique

13 patients ont été étudiés pour évaluer le nombre de leurs lymphocytes circulant identifiés comme étant des cellules T auxiliaires (helper) (CD4+) et le rapport de : cellules T auxiliaires/cellules T suppresseur. Parmi ces patients, 11 avaient un SIDA : leurs nombres de cellules T auxiliaires était de 243 à 300 par microlitre, et leur rapport cellules T auxiliaires/ cellules T suppresseur moyen était de 0,25 à 0,15. 1 patient présentait des signes cliniques de ARC, avait un nombre de lymphocytes T helper de 240/microlitre et un ratio de 0,18. Un autre patient, présentant une syphillis neurologique et aucun symptôme lié en façon évidente à HIV, avait un nombre de lymphocytes T helper de 690 par microlitre et un ratio de 0.82.

DISCUSSION

Dans cette étude, l'infection par HIV-2 a été mise en évidence chez 30 patients africains d'Afrique de l'Ouest présentant soit un SIDA, soit des symptômes du complexe ARC ou n'ayant aucun signe clinique rattaché au SIDA. Les résultats permettent néanmoins de conclure du fait que HIV-2 doit être considéré comme un agent éthiologique majeur du SIDA chez les patients d'Afrique de l'Ouest. Les résultats sérologiques et viraux qui ont été observés indiquent que l'infection par HIV-2 n'était pas souvent associée avec une infection par HIV-1 parmi ces patients.

En dépit de différences antigéniques et génétiques importantes, HIV-1 et HIV-2 présentent un tropisme similaire pour les lymphocytes T CD4+, ont des effets cytopatogènes similaires, une morphologie similaire, et partagent des épitopes d'immunoréaction communs pour certaines de leurs protéines constitutives.

Dans la mesure où tous les patients d'Afrique de l'Ouest, infectés avec HIV et étudiés ici ont été reconnus comme étant infectés par HIV-2 alors qu'aucun n'était infecté par HIV-1, le nouveau virus HIV-2 pourrait être la cause majeure du SIDA en Afrique de l'Ouest.

Les symptômes du SIDA liés à HIV-2 ne sont pas différents de ceux du SIDA liés à HIV-1 en Afrique Centrale : Le symptôme le plus commun consiste dans des diarrhées chroniques, avec perte importante de poids, troubles dûs la plupart du temps à Isospora belli et/ou Cryptosporidium. La fréquence d'autres infections soudaines, telles que des candidoses, des microbactéries (incluant M. tuberculosis) et des toxoplamoses comparable à ce que l'on reconnaissait chez les Africains atteints d'un SIDA lié à HIV-1. La pneumonie du type Pneumocystis carinii, considérée comme une complication très commune chez les malades atteints du SIDA aux USA et en Europe, a été décelée seulement une fois lors de notre étude, et des méningites de type cryptococcal n'ont pas été détectées.

Néanmoins, les anomalies immunologiques décelées parmi des patients atteints de SIDA lié à une infection par HIV-2 sont identiques à celles décrites lors de SIDA lié à HIV-1.

Parmi les 30 patients qui possédaient dans leur sérum des anticorps réagissant avec les antigènes HIV-2, seuls 7 d'entre-eux avaient des anticorps spécifiques contre HIV-1 détectables par des tests IFA ou ELISA. Lors d'un test RIPA, on observe que ces 7 patients présentent des anticorps réagissant contre les protéines majeures du noyau p25 (HIV-1) ou p26 (HIV-2), qui ont en commun des épitopes fortement immunoréactifs. 5 patients ne présentent pas de tels anticorps : ces 5 patients ont des réponses négatives à des tests ELISA vis-à-vis de HIV-1. 2 d'entre eux ont des réponses négatives à des tests IFA et 3 d'entre eux ont des réponses très faibles. Cependant, bien que certains d'entre eux n'aient pas été totalement étudiés, 9 patients ont été observés qui possédaient dans leur sérum des anticorps de la protéine virale p26 du noyau de HIV-2 et qui présentaient une réaction faible ou une réaction négative à des tests IFA ou ELISA spécifiques de HIV-1. Ces résultats font ressortir l'importance de l'incorporation d'antigène HIV-2 dans des tests sérologiques HIV utilisés en Afrique et peut-être dans d'autres endroits.

Un rétrovirus a été isolé des lymphocytes périphériques de 11 patients. Dans tous les cas la croissance virale a été obtenue en 2 semaines, caractérisée par la présence d'une activité de transcriptase inverse dans le surnageant de culture et par un effet cytopatogénique. Cependant, cet effet cytopatogénique est apparu d'importance variable pour les différents isolats : certains isolats présentaient un nombre important de syncytia de grande taille accompagné d'une lyse cellulaire importante, alors que d'autres présentaient seulement peu de syncytia de taille limitée, et affectant rarement la viabilité de la culture.

Tous les ARNs sauf celui d'un unique isolat hybrident avec une sonde dérivée d'un ADNc cloné de HIV-2 représentant la terminaison 3' du génome, dans des conditions de fortes stringences. Aucun n'hybride avec une sonde HIV-1 dans les mêmes conditions. Ceci démontre que les isolats infectant ces patients appartiennent au même type de virus. Un isolat seulement hybride mais rarement avec une sonde HIV-2 à la fois dans des conditions de forte et de faible stringence, et n'hybride pas avec HIV-1. Ce virus a cependant été isolé chez un patient possédant dans son sérum des anticorps capables de réagir avec tous les antigènes de HIV-2 lors d'un test RIPA.

D'une façon générale, l'invention concerne, outre le virus HIV-2 et ses variants, tout virus équivalent infectieux pour l'homme présentant des caractéristiques immunologiques propres au HIV-2. D'une façon générale, l'invention concerne tout virus qui, outre les propriétés que possède les virus HIV-2 déposés à la CNCM, présente encore les caractéristiques suivantes.

La cible préférentielle du rétrovirus HIV-2 est constituée par les cellules Leu 3 (ou lymphocytes T4) humaines et pour des cellules "immortalisées" dérivées de ces lymphocytes T4, par exemple les cellules des lignées HUT-78 considérées dans le cadre de cette demande. En d'autres termes, il a un tropisme spécifique pour ces cellules. Il peut être cultivé dans des lignées permanentes du type HUT, CEM, MOLT ou analogue exprimant la protéine T4. Il n'est pas infectieux pour les lymphocytes T8. Il est cytoxique pour les lymphocytes T4 humains. Le caractère cytopathique des virus HIV-2 à l'égard des lymphocytes T4 se manifeste notamment par l'apparition de cellules multinuclées. Il a une activité de transcriptase inverse nécessitant la présence d'ions $Mg^{2+}$ et présentant une forte affinité pour le poly(adénylate-oligodéoxythymidylase) (poly(A)-oligo (dT) 12-18). Il a une densité d'environ 1,16 dans un gradient de sucrose. Il a un diamètre moyen de 140 nanomètres et un noyau ayant un diamètre moyen de 41 nanomètres. Les lysats de ce virus contiennent une protéine p26 qui ne croise pas immunologiquement avec la protéine p24 du virus HTLV-I ou du virus HTLV-II. Ces protéines p26 présentent donc un poids moléculaire un peu plus élevée (d'environ 1000) que les p25 correspondantes des HIV-1 et un peu moins élevéed (de nouveau de l'ordre d'environ 1000) que les p27 correspondantes des SIV. Les lysats de HIV-2 contiennent en outre une protéine p16 qui n'est pas reconnue immunologiquement par la protéine p19 de HTLV-I ou de HTLV-II dans des essais de radioimmunoprécipitation RIPA (abréviation de l'expression anglaise "radioimmunoprecipitation assay"). Ils contiennent en outre une glycoprotéine d'enveloppe ayant un poids moléculaire de l'ordre de 130.000-140.000 qui ne croise pas immunologiquement avec la gp110 du HIV-1, mais qui, par contre, croise immunologiquement avec la glycoprotéine d'enveloppe gp140 de STLV-III. Ces lysats contiennent encore des protéines ou glycoprotéines, marquables par la $^{35}S$-cystéine, ayant des poids moléculaires respectivement de l'ordre de 36.000 et 42.000-45.000. L'ARN génomique de HIV-2 n'hybride pas avec l'ARN génomique de HIV-1 dans des conditions stringentes. Dans des conditions non stringentes, il n'hybride, ni avec la séquence nucléotidique dérivée de HIV-1 et contenant le gene env et le LTR qui le jouxte. En particulier il n'hybride pas avec la séquence de nucléotides 5290-9130 de HIV-1, ni avec des séquences de la région pol du génome de HIV-1, en particulier avec la séquence de nucléotides 2170-2240. Dans des conditions non stringentes, il hybride faiblement avec des séquences de nucléotides de la région de HIV-1, notamment les séquences de nucléotides 990-1070 et 990-1260.

Il est à remarquer que tout rétrovirus infectieux pour l'homme susceptible d'induire un SIDA ayant les propriétés essentielles susdites et dont l'ARN génomique est susceptible de s'hybrider dans les conditions stringentes avec ceux des souches virales de HIV-2 déposées à la C.N.C.M. (ou avec un cADN ou fragment de cADN dérivés de ces ARNs génomiques doit être considéré comme un équivalent de HIV-2.

L'invention concerne encore chacun des antigènes, notamment protéines et glycoprotéines à l'état purifié, tels qu'ils peuvent être obtenus à partir de HIV-2. On parle de protéines ou glycoprotéines "purifiées" lorsque ces protéines ou glycoprotéines ne conduisent respectivement qu'à des bandes uniques en électrophorèse sur gel de polyacrylamide, notamment dans les conditions expérimentales qui ont été indiquées plus haut. Tout procédé approprié de séparation et/ou de purification pour obtenir chacune d'entre elles peut être utilisé. On mentionnera à titre d'exemple de technique pouvant être mise en oeuvre celle décrite par R.C. MONTELARO et Coll., J. of Virology, juin 1982, pp. 1029-1038.

D'une façon générale, l'invention concerne tous antigènes, notamment protéines, glycoprotéines ou polypeptides issus d'un HIV-2 et ayant des propriétés immunologiques équivalentes à ceux ou celles du HIV-2. Deux antigènes sont dits "équivalents" dans le cadre de cet exposé, dès lors qu'ils sont reconnus par les mêmes anticorps, notamment d'anticorps isolables à partir d'un sérum obtenu à partir d'un patient ayant fait une infection avec un HIV-2, ou dès lors qu'ils répondent aux condtions "d'équivalence immunologique" indiquées ci-après.

Parmi les polypeptides, protéines ou glycoprotéines équivalents, doivent être rangés des fragments des antigènes qui précèdent (ou des ppeptides reconstitués par synthèse chimique), dès lors qu'ils donnent lieu à des réactions immunologiques croisées avec les antigènes dont ils sont dérivés. En d'autres termes, l'invention concerne tout polypeptide ayant, en commun avec les susdits antigènes, des épitopes identiques ou semblables, susceptibles d'être reconnus par les mêmes anticorps. Font partie de ce dernier type de polypeptides les produits d'expression de séquences correspondantes des ADNs codant pour les séquences polypeptidiques correspondantes.

Le virus HIV-2 s'est avéré utilisable comme source d'antigènes pour la détection d'anticorps chez toutes personnes ayant été mises en contact avec le virus HIV-2.

D'une façon générale, l'invention concerne toute composition utilisable pour le diagnostic de la présence dans un sérum fluide biologique, notamment de personnes ayant été mises au contact de HIV-2, d'anticorps contre l'un au moins des antigènes de HIV-2. Cette composition peut être appliquée au diagnostic sélectif de la variété correspondante du SIDA, en mettant en oeuvre les techniques de diagnostic, telles que décrites dans la demande de brevet européen citée ci-dessus, si ce n'est que l'on utilise des extraits, lysats ou encore antigènes purifiés de HIV-2 au lieu de ceux de HIV-I. A cet égard, l'invention concerne plus particulièrement des composition contenant l'une au moins des protéines p12, p16, p26, s'agissant des protéines internes, ou l'une au moins des glycoprotéines p36 ou gp140. On mentionnera à titre d'exemples de compositions, celles qui contiennent simultanément :
- la p26 et la gp36,
- la p26, les p36 et gp140
- les p12, p16 et p26,
- les p16, p26 et gp 140 .....

Il va de soi que ces compositions n'ont que valeur d'exemples. En particulier, l'invention concerne les extraits ou lysats viraux contenant l'ensemble de ces protéines et/ou glycoprotéines out toutes fractions dont auront au préalable été séparées une ou plusieurs des protéines ou glycoprotéines susdites.

L'invention concerne encore des compositions associant des protéines et/ou glycoprotéines d'un HIV-2, avec des protéines et/ou glycoprotéines d'un HIV-1, par exemple :
- soit des protéines du noyau (core) de HIV-1 et de HIV-2, notamment les p25 d'un HIV-1 et p26 d'un HIV-2, ou encore soit la p18 d'un HIV-1 et la p16 d'un HIV-2,
- soit des glycoprotéines d'enveloppe d'un HIV-1 et des glycoprotéines d'enveloppe d'un HIV-2, notamment la gp110 de HIV-1 et la gp140 de HIV-2, ou encore la p42 de HIV-1 et la p36 ou p42-45 de HIV-2,
- soit naturellement des mélanges de protéines et/ou glycoprotéines d'un HIV-1 et de protéines et/ou glycoprotéines d'un HIV-2.

De telles compositions, utilisées pour le diagnostic, permettent par conséquent des opérations de diagnostic du SIDA ou des symptômes qui lui sont associés, qui s'étendent sur un plus large spectre des agents étiologiques responsables. Il va sans dire que l'utilisation pour les opérations de diagnostic de compositions qui ne contiennent que des protéines et/ou glycoprotéines de HIV-2 n'en reste pas moins utile pour des diagnostics plus sélectifs de la catégorie de rétrovirus qui peut être tenue pour responsable de la maladie.

L'invention concerne encore les ADNs ou fragments d'ADNs, plus particulièrement ADNs et fragments d'ADNs clonés obtenus à partir de l'ARN ou de cADNs dérivés de l'ARN du rétrovirus HIV-2. L'invention concerne encore particulièrement tous ADNs équivalents, notamment tout ADN présentant des homologies de séquences avec l'ADN du HIV-2, en particulier avec les séquences codant pour les régions env et pol de la souche de HIV-2 déposée à la C.N.C.M., au moins égales à 50%, de préférence à 70%, et plus avantageusement encore à 90 %. D'une façon générale, on dira encore que l'invention concerne tout ADN (ou ARN) équivalent capable d'hybrider avec l'ADN ou l'ARN du HIV-2 dans la technique du "spot blot" dans des conditions non stringentes telles que définies ci-dessus.

17

L'invention concerne de la même façon les sérums susceptibles d'être produits chez l'animal par inoculation à celui-ci de HIV-2. L'invention concerne donc plus particulièrement les anticorps polyclonaux plus spécifiquement orientés contre chacun des antigènes, notamment protéines ou glycoprotéines du virus. Elle concerne aussi les anticorps monoclonaux qui peuvent être produits par des techniques classiques, ces anticorps monoclonaux étant orientés respectivement plus spécifiquement contre les différentes protéines du HIV-2.

Ces anticorps polyclonaux ou monoclonaux sont utilisables dans différentes applications. On mentionnera essentiellement leur utilisation pour neutraliser les protéines correspondantes, voir inhiber l'infectivité du virus entier. Ils peuvent également être utilisés, par exemple, pour mettre en évidence des antigènes viraux dans les préparations biologiques ou pour réaliser des opérations de purification des protéines et/ou glycoprotéines correspondantes, par exemple par leur utilisation dans des colonnes de chromatographie d'affinité.

Il est entendu que, d'une façon générale, la littérature technique disponible (notamment celle dont les références bibliographiques sont rappelées dans le cadre de la présente description) en ce qui concerne le HIV-1 et le virus dénommé HTLV-III doit être considérée comme faisant partie de la présente invention, dès lors que les techniques décrites par cette littérature s'appliquent dans des conditions semblables à l'isolement du virus HIV-2 ou de virus équivalents, à l'obtention à partir de ces virus de leurs différents constituants (notamment protéines, glycoprotéines, polypeptides, acides nucléiques). On peut également faire appel aux enseignements de cette littérature technique pour ce qui est de l'application des différents constituants concernés, notamment à des opérations de diagnostic des formes correspondantes de SLA ou de SIDA.

La présente invention concerne plus particulièrement un procédé de diagnostic in vitro du SIDA, qui comprend la mise en contact d'un sérum ou d'un autre milieu biologique provenant d'un malade faisant l'objet du diagnostic avec une composition contenant l'une au moins des protéines ou glycoprotéines du HIV-2, ou encore un extrait ou lysat du virus, et la détection de la réaction immunologique. Des exemples de telles compositions ont été indiqués plus haut.

Des méthodes préférées mettent en jeu par exemple des réactions immunoenzymatiques de type ELISA ou d'immunofluorescence. Les titrages peuvent être des mesures par immunofluorescence directe ou indirecte ou des dosages immunoenzymatiques directs ou indirects.

Ainsi la présente invention est également relative à des extraits de virus (soit d'un extrait d'un ou plusieurs HIV-2 seulement, soit d'un mélange d'extraits originaires d'un ou plusieurs HIV-2, d'une part, et d'un ou plusieurs HIV-1, d'autre part), ces extraits étant marqués. On peut utiliser tout type de marqueur approprié : enzymatique, fluorescent, radioactif, etc..

De tels titrages comprennent par exemple :
- le dépôt de quantités déterminées de l'extrait ou des compositions visées conformes à la présente invention dans les puits d'une microplaque de titrage ;
- l'introduction dans ces puits de dilutions croissantes du sérum contenant essentiellement les anticorps dont la présence doit être détectée in vitro ;
- l'incubation de la microplaque ;
- le lavage soigneux de la microplaque avc un tampon approprié ;
- l'introduction dans les puits de la microplaque d'anticorps marqués spécifiques d'immunoglobulines humaines, le marquage étant réalisé par une enzyme choisie parmi celles qui sont capables d'hydrolyser un substrat de telle sorte que ce dernier subit alors une modification de son absorption des radiations, au moins dans une bande de longueurs d'ondes déterminée et
- la détection, de préférence de façon comparative par rapport à un témoin, de l'importance de l'hydrolyse du substrat en tant que mesure des risques potentiels ou de la présence effective de la maladie.

La présente invention est également relative à des néessaires ou "kits" pour le diagnostic ci-dessus, lesquels comprennent :
- un extrait ou une fraction plus purifiée des types de virus indiqués ci-dessus, cet extrait ou fraction étant marqué, par exemple de façon radioactive, enzymatique ou immunofluorescence ;
- des anti-immunoglobulines humaines ou une protéine A (fixée de façon avantageuse sur un support insoluble dans l'eau, tel que des billes d'agarose) ;
- un extrait de lymphocytes obtenus à partir d'une personne en bonne santé ;
- des tampons et, le cas échéant, des substrats pour la visualisation du marquage.

De ce qui précède, il résulte que l'invention concerne le diagnostic du virus HIV-2 ou d'un variant grâce à la mise en oeuvre des sondes décrites précédemment dans un procédé faisant appel à différentes étapes rappelées ci-après, ces étapes étant spécifiquement prévues pour faire apparaître les propriétés caractéristiques du virus HIV-2.

L'invention concerne naturellement aussi l'utilisation des cADNs ou de leurs fragments (ou de recombinants les contenant) en tant que sondes, pour le diagnostic de la présence ou non de virus HIV-2 dans des échantillons de sérums ou d'autres liquides ou tissus biologiques obtenus à partir de patients suspectés d'être porteurs du virus HIV-2. Ces sondes sont de préférence marquées également (marqueurs radio-actifs, enzymatiques, fluorescents, etc.). Des sondes particulièrement interessantes pour la mise en oeuvre du procédé de diagnostic du virus HIV-2 ou d'un variant de HIV-2 peuvent être caractérisées en ce qu'elles comprennent la totalité ou une fraction de l'ADNc complémentaire du génome du virus HIV-2 ou encore notamment les fragments contenus dans les divers clones identifiés ci-dessus. On mentionnera plus particulièrement une fraction de l'ADNc de HIV-2 contenu dans le clone E2, plus particulièrement la séquence

**0 269 520**

de l'extrémité 3' (LTR) et/ou de l'extrémité 5' de la séquence HIV du clone E2 précité, ou encore l'ADNc contenant la région env, de l'ADNc du virus HIV-2.

Les sondes mises en oeuvre dans ce procédé de diagnostic du virus HIV-2 et dans les kits de diagnostic, ne sont en aucune façon réduites aux sondes décrites précédemment. Elles comprennent au contraire toutes les séquences nucléotidiques issues de génome du virus HIV-2, d'un variant de HIV-2 ou d'un virus proche par sa structure, dès lors qu'elles permettent la détection dans des fluides biologiques de personnes susceptibles de développer un SIDA d'anticorps dirigés contre un HIV-2. Naturellement l'utilisation de séquences nucléotidiques issues d'un HIV-2, initialement infectieux pour l'homme, est néanmoins préférée.

La détection peut être réalisée de toutes façons en soi connues, notamment par une mise en contact de ces sondes soit avec les acides nucléiques obtenus à partir des cellules contenues dans ces sérums ou autres milieux biologiques, par exemple liquides céphalo-rachidiens, salive, etc.., soit avec ces milieux eux-mêmes dès lors que leurs acides nucléiques auront été rendus accessibles à l'hybridation avec ces sondes, et ce dans des conditions permettant l'hybridation entre ces sondes et ces acides nucléiques et par une détection de l'hybridation éventuellement produite. Le susdit diagnostic mettant en jeu des réactions d'hybridation peut également être réalisé à l'aide de mélanges de sondes respectivement originaires d'un HIV-1 et d'un HIV-2, dès lors qu'il n'est pas nécessaire de faire une différence entre le type de virus HIV recherché.

D'une façon générale, le procédé de diagnostic de la présence ou non du virus HIV-2 ou d'un variant dans des échantillons de sérums ou d'autres liquides ou tissus obtenus à partir de patients suspectés d'être porteurs du virus HIV-2 comprend les étapes suivantes :

1) la fabrication d'un sonde marquée,

2) au moins une étape d'hybridation conduite dans des conditions stringentes, par mise en contact de l'ADN de cellules de l'échantillon du patient suspect avec ladite sonde marquée sur une menmbrane appropriée,

3) le lavage de ladite membrane avec une solution assurant la conservation de ces conditions stringentes de l'hybridation,

4) la détection de la présence ou non du virus HIV-2 par une méthode d'immunodétection.

Dans un autre mode de réalisation préféré du procédé selon l'invention l'hybridation précitée est conduite dans des conditions non stringentes et le lavage de la membrane est réalisé dans des conditions adaptées à celles de l'hybridation.

L'invention concerne en particulier les virus HIV-2, caractérisés par le fait que leur ARN viral est en correspondance avec un ADNc dont les régions contenant les gènes gag et env comportent respectivement les séquences nucléotidiques qui suivent (GAGRODN et ENVRN). Elles résultent du séquençage des régions correspondantes de l'ADNc correspondant au génome de HIV-2 ROD. Elles sont mises en correspondance avec les acides aminés qu'elles codent.

GAGRODN

```
MetGlyAlaArgAsnSerValLeuArgGlyLysLysAlaAspGlu
ATGGGCGCGAGAAACTCCGTCTTGAGAGGGAAAAAAGCAGATGAA

LeuGluArgIleArgLeuArgProGlyGlyLysLysLysTyrArg
TTAGAAAGAATCAGGTTACGGCCCGGCGGAAAGAAAAAGTACAGG

LeuLysHisIleValTrpAlaAlaAsnLysLeuAspArgPheGly
CTAAAACATATTGTGTGGGCAGCGAATAAATTGGACAGATTCGGA
          100
LeuAlaGluSerLeuLeuGluSerLysGluGlyCysGlnLysIle
TTAGCAGAGAGCCTGTTGGAGTCAAAAGAGGGTTGTCAAAAAATT

LeuThrValLeuAspProMetValProThrGlySerGluAsnLeu
CTTACAGTTTTAGATCCAATGGTACCGACAGGTTCAGAAAATTTA
                    200
LysSerLeuPheAsnThrValCysValIleTrpCysIleHisAla
AAAAGTCTTTTTAATACTGTCTGCGTCATTTGGTGCATACACGCA

GluGluLysValLysAspThrGluGlyAlaLysGlnIleValArg
GAAGAGAAAGTGAAAGATACTGAAGGAGCAAAACAAATAGTGCGG
                              300
ArgHisLeuValAlaGluThrGlyThrAlaGluLysMetProSer
AGACATCTAGTGGCAGAAACAGGAACTGCAGAGAAAATGCCAAGC

ThrSerArgProThrAlaProSerSerGluLysGlyGlyAsnTyr
ACAAGTAGACCAACAGCACCATCTAGCGAGAAGGGAGGAAATTAC
                                      400
ProValGlnHisValGlyGlyAsnTyrThrHisIleProLeuSer
CCAGTGCAACATGTAGGCGGCAACTACACCCATATACCGCTGAGT

ProArgThrLeuAsnAlaTrpValLysLeuValGluGluLysLys
CCCCGAACCCTAAATGCCTGGGTAAAATTAGTAGAGGAAAAAAAG

PheGlyAlaGluValValProGlyPheGlnAlaLeuSerGluGly
TTCGGGGCAGAAGTAGTGCCAGGATTTCAGGCACTCTCAGAAGGC
500
CysThrProTyrAspIleAsnGlnMetLeuAsnCysValGlyAsp
TGCACGCCCTATGATATCAACCAAATGCTTAATTGTGTGGGCGAC

HisGlnAlaAlaMetGlnIleIleArgGluIleIleAsnGluGlu
CATCAAGCAGCCATGCAGATAATCAGGGAGATTATCAATGAGGAA
                600
AlaAlaGluTrpAspValGlnHisProIleProGlyProLeuPro
GCAGCAGAATGGGATGTGCAACATCCAATACCAGGCCCCTTACCA

AlaGlyGlnLeuArgGluProArgGlySerAspIleAlaGlyThr
GCGGGGCAGCTTAGAGAGCCAAGGGGATCTGACATAGCAGGGACA
                    700
ThrSerThrValGluGluGlnIleGlnTrpMetPheArgProGln
ACAAGCACAGTAGAAGAACAGATCCAGTGGATGTTTAGGCCACAA
```

```
AsnProValProValGlyAsnIleTyrArgArgTrpIleGlnIle
AATCCTGTACCAGTAGGAAACATCTATAGAAGATGGATCCAGATA
                             800
GlyLeuGlnLysCysValArgMetTyrAsnProThrAsnIleLeu
GGATTGCAGAAGTGTGTCAGGATGTACAACCCGACCAACATCCTA

AspIleLysGlnGlyProLysGluProPheGlnSerTyrValAsp
GACATAAAACAGGGACCAAAGGAGCCGTTCCAAAGCTATGTAGAT
                                   900
ArgPheTyrLysSerLeuArgAlaGluGlnThrAspProAlaVal
AGATTCTACAAAGCTTGAGGGCAGAACAAACAGATCCAGCAGTG

LysAsnTrpMetThrGlnThrLeuLeuValGlnAsnAlaAsnPro
AAGAATTGGATGACCCAAACACTGCTAGTACAAAATGCCAACCCA

AspCysLysLeuValLeuLysGlyLeuGlyMetAsnProThrLeu
GACTGTAAATTAGTGCTAAAAGGACTAGGGATGAACCCTACCTTA
      1000
GluGluMetLeuThrAlaCysGlnGlyValGlyGlyProGlyGln
GAAGAGATGCTGACCGCCTGTCAGGGGGTAGGTGGGCCAGGCCAG

LysAlaArgLeuMetAlaGluAlaLeuLysGluValIleGlyPro
AAAGCTAGATTAATGGCAGAGGCCCTGAAAGAGGTCATAGGACCT
                1100
AlaProIleProPheAlaAlaAlaGlnGlnArgLysAlaPheLys
GCCCCTATCCCATTCGCAGCAGCCCAGCAGAGAAAGGCATTTAAA

CysTrpAsnCysGlyLysGluGlyHisSerAlaArgGlnCysArg
TGCTGGAACTGTGGAAAGGAAGGGCACTCGGCAAGACAATGCCGA
                     1200
AlaProArgArgGlnGlyCysTrpLysCysGlyLysProGlyHis
GCACCTAGAAGGCAGGGCTGCTGGAAGTGTGGTAAGCCAGGACAC

IleMetThrAsnCysProAspArgGlnAlaGlyPheLeuGlyLeu
ATCATGACAAACTGCCCAGATAGACAGGCAGGTTTTTTAGGACTG
                                   1300
GlyProTrpGlyLysLysProArgAsnPheProValAlaGlnVal
GGCCCTTGGGGAAAGAAGCCCCGCAACTTCCCCGTGGCCCAAGTT

ProGlnGlyLeuThrProThrAlaProProValAspProAlaVal
CCGCAGGGGCTGACACCAACAGCACCCCCAGTGGATCCAGCAGTG

AspLeuLeuGluLysTyrMetGlnGlnGlyLysArgGlnArgGlu
GATCTACTGGAGAAATATATGCAGCAAGGGAAAAGACAGAGAGAG
  1400
GlnArgGluArgProTyrLysGluValThrGluAspLeuLeuHis
CAGAGAGAGAGACCATACAAGGAAGTGACAGAGGACTTACTGCAC

LeuGluGlnGlyGluThrProTyrArgGluProProThrGluAsp
CTCGAGCAGGGGGAGACACCATACAGGGAGCCACCAACAGAGGAC
              1500
LeuLeuHisLeuAsnSerLeuPheGlyLysAspGln
TTGCTGCACCTCAATTCTCTCTTTGGAAAAGACCAG
```

21

ENVRN

```
MetMetAsnGlnLeuLeuIleAlaIleLeuLeuAlaSerAlaCys
ATGATGAATCAGCTGCTTATTGCCATTTTATTAGCTAGTGCTTGC

LeuValTyrCysThrGlnTyrValThrValPheTyrGlyValPro
TTAGTATATTGCACCCAATATGTAACTGTTTTCTATGGCGTACCC

ThrTrpLysAsnAlaThrIleProLeuPheCysAlaThrArgAsn
ACGTGGAAAAATGCAACCATTCCCCTCTTTTGTGCAACCAGAAAT
         100
ArgAspThrTrpGlyThrIleGlnCysLeuProAspAsnAspAsp
AGGGATACTTGGGGAACCATACAGTGCTTGCCTGACAATGATGAT

TyrGlnGluIleThrLeuAsnValThrGluAlaPheAspAlaTrp
TATCAGGAAATAACTTTGAATGTAACAGAGGCTTTTGATGCATGG
                 200
AsnAsnThrValThrGluGlnAlaIleGluAspValTrpHisLeu
AATAATACAGTAACAGAACAAGCAATAGAAGATGTCTGGCATCTA

PheGluThrSerIleLysProCysValLysLeuThrProLeuCys
TTCGAGACATCAATAAAACCATGTGTCAAACTAACACCTTTATGT
                         300
ValAlaMetLysCysSerSerThrGluSerSerThrGlyAsnAsn
GTAGCAATGAAATGCAGCAGCACAGAGAGCAGCACAGGGAACAAC

ThrThrSerLysSerThrSerThrThrThrThrThrProThrAsp
ACAACCTCAAAGAGCACAAGCACAACCACAACCACACCCACAGAC
                                 400
GlnGluGlnGluIleSerGluAspThrProCysAlaArgAlaAsp
CAGGAGCAAGAGATAAGTGAGGATACTCCATGCGCACGCGCAGAC

AsnCysSerGlyLeuGlyGluGluGluThrIleAsnCysGlnPhe
AACTGCTCAGGATTGGGAGAGGAAGAAACGATCAATTGCCAGTTC

AsnMetThrGlyLeuGluArgAspLysLysLysGlnTyrAsnGlu
AATATGACAGGATTAGAAAGAGATAAGAAAAAACAGTATAATGAA
500
ThrTrpTyrSerLysAspValValCysGluThrAsnAsnSerThr
ACATGGTACTCAAAAGATGTGGTTTGTGAGACAAATAATAGCACA

AsnGlnThrGlnCysTyrMetAsnHisCysAsnThrSerValIle
AATCAGACCCAGTGTTACATGAACCATTGCAACACATCAGTCATC
       600
ThrGluSerCysAspLysHisTyrTrpAspAlaIleArgPheArg
ACAGAATCATGTGACAAGCACTATTGGGATGCTATAAGGTTTAGA

TyrCysAlaProProGlyTyrAlaLeuLeuArgCysAsnAspThr
TACTGTGCACCACCGGGTTATGCCCTATTAAGATGTAATGATACC
                 700
AsnTyrSerGlyPheAlaProAsnCysSerLysValValAlaSer
AATTATTCAGGCTTTGCACCCAACTGTTCTAAAGTAGTAGCTTCT
```

```
ThrCysThrArgMetMetGluThrGlnThrSerThrTrpPheGly
ACATGCACCAGGATGATGGAAACGCAAACTTCCACATGGTTTGGC
                            800
PheAsnGlyThrArgAlaGluAsnArgThrTyrIleTyrTrpHis
TTTAATGGCACTAGAGCAGAGAATAGAACATATATCTATTGGCAT

GlyArgAspAsnArgThrIleIleSerLeuAsnLysTyrTyrAsn
GGCAGAGATAATAGAACTATCATCAGCTTAAACAAATATTATAAT
                            900
LeuSerLeuHisCysLysArgProGlyAsnLysThrValLysGln
CTCAGTTTGCATTGTAAGAGGCCAGGGAATAAGACAGTGAAACAA

IleMetLeuMetSerGlyHisValPheHisSerHisTyrGlnPro
ATAATGCTTATGTCAGGACATGTGTTTCACTCCCACTACCAGCCG

IleAsnLysArgProArgGlnAlaTrpCysTrpPheLysGlyLys
ATCAATAAAAGACCCAGACAAGCATGGTGCTGGTTCAAAGGCAAA
      1000
TrpLysAspAlaMetGlnGluValLysThrLeuAlaLysHisPro
TGGAAAGACGCCATGCAGGAGGTGAAGACCCTTGCAAAACATCCC

ArgTyrArgGlyThrAsnAspThrArgAsnIleSerPheAlaAla
AGGTATAGAGGAACCAATGACACAAGGAATATTAGCTTTGCAGCG
              1100
ProGlyLysGlySerAspProGluValAlaTyrMetTrpThrAsn
CCAGGAAAAGGCTCAGACCCAGAAGTAGCATACATGTGGACTAAC

CysArgGlyGluPheLeuTyrCysAsnMetThrTrpPheLeuAsn
TGCAGAGGAGAGTTTCTCTACTGCAACATGACTTGGTTCCTCAAT
                      1200
TrpIleGluAsnLysThrHisArgAsnTyrAlaProCysHisIle
TGGATAGAGAATAAGACACACCGCAATTATGCACCGTGCCATATA

LysGlnIleIleAsnThrTrpHisLysValGlyArgAsnValTyr
AAGCAAATAATTAACACATGGCATAAGGTAGGGAGAAATGTATAT
                            1300
LeuProProArgGluGlyGluLeuSerCysAsnSerThrValThr
TTGCCTCCCAGGGAAGGGGAGCTGTCCTGCAACTCAACAGTAACC

SerIleIleAlaAsnIleAspTrpGlnAsnAsnAsnGlnThrAsn
AGCATAATTGCTAACATTGACTGGCAAAACAATAATCAGACAAAC

IleThrPheSerAlaGluValAlaGluLeuTyrArgLeuGluLeu
ATTACCTTTAGTGCAGAGGTGGCAGAACTATACAGATTGGAGTTG
 1400
GlyAspTyrLysLeuValGluIleThrProIleGlyPheAlaPro
GGAGATTATAAATTGGTAGAAATAACACCAATTGGCTTCGCACCT
```

ThrLysGluLysArgTyrSerSerAlaHisGlyArgHisThrArg
ACAAAGAAAAAGATACTCCTCTGCTCACGGGAGACATACAAGA
1500

GlyValPheValLeuGlyPheLeuGlyPheLeuAlaThrAlaGly
GGTGTGTTCGTGCTAGGGTTCTTGGGTTTTCTCGCAACAGCAGGT

SerAlaMetGlyAlaArgAlaSerLeuThrValSerAlaGlnSer
TCTGCAATGGGCGCTCGAGCGTCCCTGACCGTGTCGGCTCAGTCC
1600

ArgThrLeuLeuAlaGlyIleValGlnGlnGlnGlnGlnLeuLeu
CGGACTTTACTGGCCGGGATAGTGCAGCAACAGCAACAGCTGTTG

AspValValLysArgGlnGlnGluLeuLeuArgLeuThrValTrp
GACGTGGTCAAGAGACAACAAGAACTGTTGCGACTGACCGTCTGG
1700

GlyThrLysAsnLeuGlnAlaArgValThrAlaIleGluLysTyr
GGAACGAAAAACCTCCAGGCAAGAGTCACTGCTATAGAGAAGTAC

LeuGlnAspGlnAlaArgLeuAsnSerTrpGlyCysAlaPheArg
CTACAGGACCAGGCGCGGCTAAATTCATGGGGATGTGCGTTTAGA
1800

GlnValCysHisThrThrValProTrpValAsnAspSerLeuAla
CAAGTCTGCCACACTACTGTACCATGGGTTAATGATTCCTTAGCA

ProAspTrpAspAsnMetThrTrpGlnGluTrpGluLysGlnVal
CCTGACTGGGACAATATGACGTGGCAGGAATGGGAAAAACAAGTC

ArgTyrLeuGluAlaAsnIleSerLysSerLeuGluGlnAlaGln
CGCTACCTGGAGGCAAATATCAGTAAAAGTTTAGAACAGGCACAA
1900

IleGlnGlnGluLysAsnMetTyrGluLeuGlnLysLeuAsnSer
ATTCAGCAAGAGAAAAATATGTATGAACTACAAAAATTAAATAGC

TrpAspIlePheGlyAsnTrpPheAspLeuThrSerTrpValLys
TGGGATATTTTTGGCAATTGGTTTGACTTAACCTCCTGGGTCAAG
2000

TyrIleGlnTyrGlyValLeuIleIleValAlaValIleAlaLeu
TATATTCAATATGGAGTGCTTATAATAGTAGCAGTAATAGCTTTA

ArgIleValIleTyrValValGlnMetLeuSerArgLeuArgLys
AGAATAGTGATATATGTAGTACAAATGTTAAGTAGGCTTAGAAAG

GlyTyrArgProValPheSerSerProProGlyTyrIleGlnGln
GGCTATAGGCCTGTTTTCTCTTCCCCCCCCGGTTATATCCAACAG
2100

```
IleHisIleHisLysAspArgGlyGlnProAlaAsnGluGluThr
ATCCATATCCACAAGGACCGGGGACAGCCAGCCAACGAAGAAACA
                    .           .           .           2200
GluGluAspGlyGlySerAsnGlyGlyAspArgTyrTrpProTrp
GAAGAAGACGGTGGAAGCAACGGTGGAGACAGATACTGGCCCTGG
        .           .           .           .           .           .
ProIleAlaTyrIleHisPheLeuIleArgGlnLeuIleArgLeu
GCGATAGCATATATACATTTCCTGATCCGCCAGCTGATTCGCCTC
        .           .           .           .           .           .
LeuThrArgLeuTyrSerIleCysArgAspLeuLeuSerArgSer
TTGACCAGACTATACAGCATCTGCAGGGACTTACTATCCAGGAGC
    2300              .           .           .           .
PheLeuThrLeuGlnLeuIleTyrGlnAsnLeuArgAspTrpLeu
TTCCTGACCCTCCAACTCATCTACCAGAATCTCAGAGACTGGCTG
        .           .           .           .           .
ArgLeuArgThrAlaPheLeuGlnTyrGlyCysGluTrpIleGln
AGACTTAGAACAGCCTTCTTGCAATATGGGTGCGAGTGGATCCAA
        .           2400              .           .           .
GluAlaPheGlnAlaAlaAlaArgAlaThrArgGluThrLeuAla
GAAGCATTCCAGGCCGCCGCGAGGGCTACAAGAGAGACTCTTGCG
        .           .           .           .           .       .
GlyAlaCysArgGlyLeuTrpArgValLeuGluArgIleGlyArg
GGCGCGTGCAGGGGCTTGTGGAGGGTATTGGAACGAATCGGGAGG
        .           .           2500              .           .
GlyIleLeuAlaValProArgArgIleArgGlnGlyAlaGluIle
GGAATACTCGCGGTTCCAAGAAGGATCAGACAGGGAGCAGAAATC
    . :               .           .           .           .
AlaLeuLeu***GlyThrAlaValSerAlaGlyArgLeuTyrGlu
GCCCTCCTGTGAGGGACGGCAGTATCAGCAGGGAGACTTTATGAA
        .           .           .           2600              .
TyrSerMetGluGlyProSerSerArgLysGlyGluLysPheVal
TACTCCATGGAAGGACCCAGCAGCAGAAAGGGAGAAAAATTTGTA
        .           .           .           .           .
GlnAlaThrLysTyrGly
CAGGCAACAAAATATGGA
        .           .
```

Comme déjà indiqué plus haut, l'invention concerne naturellement tous les HIV-2 dont les ARNs présentent des caractéristiques semblables, en particulier des régions gag et env comportant des séquences ayant des homologies de séquence nucléotidique d'au moins 50%, de préférence 70% et plus avantageusement encore 90% avec les séquences gag et env de HIV-2 ROD.

L'invention concerne plus particulièrement les fragments d'ADNc qui codent respectivement pour les protéines p16, p26 et p12 et qui sont également inclues dans GAGRODN. En particulier elle concerne les séquences s'étendant :
- à partir du nucléotide 1 jusqu'au nucléotide 405 (codant pour p16)
- à partir du nucléotide 406 jusqu'au nucléotide 1155 (codant pour p26) et,
- à partir du nucléotide 1156 jusqu'au nucléotide 1566 (codant pour p12).

Elle concerne aussi plus particulièrement le fragment d'ADNc codant pour la gp140 inclue dans ENVRN et s'étendant à partir du nucléotide 1 jusqu'au nucléotide 2574.

Elle concerne également les séquences de nucléotides qui se distinguent des précédentes par des substitutions de nucléotides mettant à profit la dégénérescence du code génétique, dès lors que ces substitutions n'entraînent pas une modification des séquences en acides aminés que codent lesdites séquences de nucléotides.

De même l'invention concerne les protéines ou glycoprotéines dont les séquences en acides aminés correspondent à celles qui découlent des pages précédentes, ainsi que les peptides équivalents à savoir des peptides se distinguant de ceux qui découlent des pages précédentes par addition, substitution ou délétion d'acides aminés qui n'affectent pas les propriétés immunologiques globales desdits peptides.

L'invention concerne tout particulièrement la glycoprotéine d'enveloppe présentant la séquence en acides aminés qui découle de ENVRN.

0 269 520

L'invention concerne également une composition immunogène caractérisée en ce qu'elle est dosée en antigène, tel que la gp 140 du virus HIV-2, de façon à permettre l'administration de doses unitaires de 10 à 500, notamment du 50 à 100 mg par kg de corps.

Enfin l'invention concerne un procédé de fabrication de l'une des protéines susdéfinies (p12, p16 ou p26) ou d'une protéine ayant la structure de gp140, ou de parties déterminées de ces protéines, ce procédé étant caractérisé par l'insertion de la séquence d'acide nucléique correspondante dans un vecteur susceptible de transformer un hôte cellulaire choisi et de permettre l'expression d'un insérat contenu dans ce vecteur, par la transformation de cet hôte choisi par ledit vecteur contenant ladite séquence nucléique, par la culture de l'hôte cellulaire transformé et par la récupération et la purification de la protéine exprimée.

Les techniques décrites dans la demande de brevet européen 85.905.513 9 déposée le 18 Octobre 1985 pour la production de peptides ou de protéines consistant en des produits d'expression de séquences d'acides nucléiques dérivées du génome de HIV-1 sont applicables également à la production des susdits peptides ou protéines dérivés de HIV-2. La description de cette demande de brevet européen doit être considérée comme faisant partie de la présente demande en ce qui concerne les techniques.

A titre indicatif, les poids moléculaires théoriques (PM) des protéines de HIV-2 sont indiqués, en comparaison avec ceux de HIV-1.

| PM des protéines de HIV-2 en kd | | PM des protéines pour HIV-1 en kd | |
|---|---|---|---|
| gag complet | 58,3 | gag complet | 55,8 |
| p 16 | 15 | p 18 | 14,9 |
| p 26 | 27,6 | | |
| p 12 | 15,8 | | |
| env | 98,6 | env | 97,4 |
| env externe | 57,4 | | |
| env transmembranaire | 41,2 | | |

HIV-2 MIR et HIV-2 ROD ont également été déposés à la "National Collection of Animal Cell Cultures" (ECACC) à SALISBURY (Grande Bretagne) le 9 Janvier 1987 sous les numéros d'accès 87. 01.1001 et 87. 01.1002 respectivement.

De plus, les plasmides pROD35 et pROD27,5 ont été déposés à la "National Collection of Industrial Bacteria" (NCIB) à ABERDEEN (Grande Bretagne) le 9 Janvier 1987 sous les numéros d'accès respectifs 12.398 et 12.399.

Toutes les publications auxquelles il a été fait référence dans cette description doivent être considérées comme faisant partie de cette description.

BIBLIOGRAPHIE

1 - F. Barré-Sinoussi et al., Science 220, 868 (1983).
2 - L. Montagnier et al., In : Human Tcell leukemia Orlymphoma viruses (Gallo, R.C., Essex, M.E., Gross, L., eds.) Cold Spring Harbor Laboratory, New York, 363 (1984).
3 - M. Popovic, M.G. Sarngadharan, E. Read, R.C. Gallo, Science 224, 497 (1984).
4 - J. Levy et al., Science 225, 840 (1984).
5 - P. Sonigo et al., Cell 42, 369 (1985).
7 - J.W. Curran et al., Science 229, 1352 (1985).
8 - A. Ellrodt et al., Lancet i, 1383 (1984).
9 - P. Piot et al., Lancet ii, 65 (1984).
10 - F. Brun-Vezinet et al., Science 226, 453 (1984).
11 - N. Clumeck et al., N. Engl. J. Med. 313, 182 (1985).
12 - A.B. Rabson and M.A. Martin, Cell 40, 477 (1985).
13 - S. Benn et al., Science 230, 949 (1985).
14 - M. Alizon, Manuscript in preparation.
15 - F. Brun-Vezinet, Unpublished data.
16 - M.D. Daniel et al., Science 228, 1201 (1985).
17 - P.J. Kanki et al., Science 228, 1199 (1985).
18 - N.L. Letwin et al., Science 230, 71 (1985).

19 - A. Gatzar et al., Blood 55, 409 (1980).

20 - D. Klatzmann et al., Science 225, 59 (1984).

21 - L. Montagnier et al., Virology 144, 283 (1985).

22 - J.S. Allan et al., Science 228, 1091 (1985).

23 - F. Chivel, Manuscript in preparation.

24 - F. DiMarzo Veronese et al., Science 229, 1402 (1985).

25 - V.S. Kalyanaraman et al., Science 218, 571 (1982).

26 - I.S.Y. Chen, J. McLaughlin, J.C. Gasson, S.C. Clark and D.W. Golde, Nature 305, 502 (1983).

27 - F. Barin et al., Lancet ii, 1387 (1985).

28 - P.J. Kanki, J. Alroy, M. Essex, Science 230, 951 (1985).

29 - H. Towbin et al., Proc. Natl. Acad. Sci. USA 76, 4350 (1979).

30 - S. Wain-Hobson, P. Sonigo, O. Danos, S. Cole et M. Alizon, Cell 40, 9 (1985).

31 - M. Alizon et al., Nature 312, 757 (1984).

## Revendications

1 - Rétrovirus HIV-2 ou des variants de ce virus, ces rétrovirus ayant des propriétés infectieuses vis-à-vis des lymphocytes T4 humains, et les propriétés morphologiques et immunologiques essentielles de l'un des rétrovirus déposés à la CNCM sous les no I-502, I-532, I-642 et I-643.

2 - Rétrovirus purifié selon la revendication 1, caractérisé par les propriétés suivantes :
- la cible préférentielle du rétrovirus HIV-2 est constituée par les cellules Leu3 (ou lymphocytes T4) humaines et pour des lignées cellulaires permanentes dérivées de ces lymphocytes T4 ;
- il est cytotoxique pour les lymphocytes T4 humains qu'il infecte ;
- il a une activité de transcriptase inverse nécessitant la présence d'ions $Mg^{2+}$ et présentant une forte activité pour le poly(adénylate-oligodéoxythylmidylase) (poly(A)-oligo(DT) 12-18) ;
- il a une densité d'environ 1,16 dans un gradient de sucrose ;
- il a un diamètre moyen de 140 nanomètres et un noyau ayant un diamètre moyen de 41 nanomètres ;
- il peut être cultivé dans des lignées permanentes exprimant la protéine T4 ;
- il n'est pas infectieux pour les lymphocytes T8 ;
- les lysats de ce virus contiennent une protéine p26 qui ne croise pas immunologiquement avec la protéine p24 du virus HTLV-1 ou du virus HTLV-2 ;
- ces lysats contiennent en outre une protéine p16 qui n'est pas reconnue immunologiquement par la protéine p19 de HTLV-1 ou de HTLV-2 dans des essais de radioimmuno-précipitation ;
- ils contiennent en outre une glycoprotéine d'enveloppe ayant un poids moléculaire de l'ordre de 130.000-140.000 qui ne croise pas immunologiquement avec la gp110 du rétrovirus HIV-1 ;
- ces lysats contiennent encore une molécule, marquable par la $^{35}$S-cystéine, ayant un poids moléculaire apparent de l'ordre de 36.000 ;
- l'ARN génomique de HIV-2 n'hybride ni avec l'ARN génomique, ni avec le gène env, ni avec les LTR de HIV-1 de HIV-1 dans des conditions stringentes ;
- l'ARN génomique de HIV-2 hybride faiblement dans des conditions non stringentes avec des séquences de nucléotides de la région gag du génome de HIV-1.

3 - Rétrovirus selon la revendication 2, caractérisé en ce que des lysats de ce rétrovirus contiennent également une molécule ayant un poids moléculaire apparent de l'ordre de 42.000-45.000.

4 - Rétrovirus selon l'une quelconque des revendications 1 à 3, caractérisé en ce que la séquence comprenant la région R et la région U3 de la séquence comprenant son ARN génomique contient une séquence nucléotidique en correspondance avec la séquence de nucléotides :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA

5 - Rétrovirus selon l'une quelconque des revendications 1 à 4, caractérisé en ce que son ARN génomique contient une séquence gag en correspondance avec la séquence de nucléotides :

GAGRODII

ATGGGCGCGAGAAACTCCGTCTTGAGAGGGAAAAAAGCAGATGAA

TTAGAAAGAATCAGGTTACGGCCCGGCGGAAAGAAAAAGTACAGG

CTAAAACATATTGTGTGGGCAGCGAATAAATTGGACAGATTCGCA
100

TTAGCAGAGAGCCTGTTGGAGTCAAAAGAGGGTTGTCAAAAAATT

CTTACAGTTTTAGATCCAATGGTACCGACAGGTTCAGAAAATTTA
200

AAAAGTCTTTTTAATACTGTCTGCGTCATTTGGTGCATACACGCA

GAAGAGAAAGTGAAAGATACTGAAGGAGCAAAACAAATAGTGCGG
300

AGACATCTAGTGGCAGAAACAGGAACTGCAGAGAAAATGCCAAGC

ACAAGTAGACCAACAGCACCATCTAGCGAGAAGGGAGGAAATTAC
400

CCAGTGCAACATGTAGGCGGCAACTACACCCATATACCGCTGAGT

CCCCGAACCCTAAATGCCTGGGTAAAATTAGTAGAGGAAAAAAAG

TTCGGGGCAGAAGTAGTGCCAGGATTTCAGGCACTCTCAGAAGGC
500

TGCACGCCCTATGATATCAACCAAATGCTTAATTGTGTGGGCGAC

CATCAAGCAGCCATGCAGATAATCAGGGAGATTATCAATGAGGAA
600

GCAGCAGAATGGGATGTGCAACATCCAATACCAGGCCCCTTACCA

GCGGGGCAGCTTAGAGAGCCAAGGGGATCTGACATAGCAGGGACA
700

ACAAGCACAGTAGAAGAACAGATCCAGTGGATGTTTAGGCCACAA

AATCCTGTACCAGTAGGAAACATCTATAGAAGATGGATCCAGATA
800

GGATTGCAGAAGTGTGTCAGGATGTACAACCCGACCAACATCCTA

GACATAAAACAGGGACCAAAGGAGCCGTTCCAAAGCTATGTAGAT
900

AGATTCTACAAAAGCTTGAGGGCAGAACAAACAGATCCAGCAGTG

AAGAATTGGATGACCCAAACACTGCTAGTACAAAATGCCAACCCA

GACTGTAAATTAGTGCTAAAAGGACTAGGGATGAACCCTACCTTA
1000

GAAGAGATGCTGACCGCCTGTCAGGGGGTAGGTGGGCCAGGCCAG

AAAGCTAGATTAATGGCAGAGGCCCTGAAAGAGGTCATAGGACCT
1100

GCCCCTATCCCATTCGCAGCAGCCCAGCAGAGAAAGGCATTTAAA

TGCTGGAACTGTGGAAAGGAAGGGCACTCGGCAAGACAATGCCGA
1200

GCACCTAGAAGGCAGGGCTGCTGGAAGTGTGGTAAGCCAGGACAC

ATCATGACAAACTGCCCAGATAGACAGGCAGGTTTTTTAGGACTG
1300

GGCCCTTGGGGAAAGAAGCCCCGCAACTTCCCCGTGGCCCAAGTT

CCGCAGGGGCTGACACCAACAGCACCCCCAGTGGATCCAGCAGTG

GATCTACTGGAGAAATATATGCAGCAAGGGAAAAGACAGAGAGAG
1400

CAGAGAGAGAGACCATACAAGGAAGTGACAGAGGACTTACTGCAC·

CTCGAGCAGGGGGAGACACCATACAGGGAGCCACCAACAGAGGAC
1500

TTGCTGCACCTCAATTCTCTCTTTGGAAAAGACCAG

6 - Rétrovirus selon l'une quelconque des revendications 1 à 5, caractérisé en ce que son ARN génomique contient une séquence env en correspondance avec la séquence de nucléotides :

ENVRN

ATGATGAATCAGCTGCTTATTGCCATTTTATTAGCTAGTGCTTGC

TTAGTATATTGCACCCAATATGTAACTGTTTTCTATGGCGTACCC

ACGTGGAAAAATGCAACCATTCCCCTCTTTTGTGCAACCAGAAAT
                100

AGGGATACTTGGGGAACCATACAGTGCTTGCCTGACAATGATGAT

TATCAGGAAATAACTTTGAATGTAACAGAGGCTTTTGATGCATGG
                200

AATAATACAGTAACAGAACAAGCAATAGAAGATGTCTGGCATCTA

TTCGAGACATCAATAAAACCATGTGTCAAACTAACACCTTTATGT
                        300

GTAGCAATGAAATGCAGCAGCACAGAGAGCAGCACAGGGAACAAC

ACAACCTCAAAGAGCACAAGCACAACCACAACCACACCCACAGAC
                                    400

CAGGAGCAAGAGATAAGTGAGGATACTCCATGCGCACGCGCAGAC

AACTGCTCAGGATTGGGAGAGGAAGAAACGATCAATTGCCAGTTC

AATATGACAGGATTAGAAAGAGATAAGAAAAAACAGTATAATGAA
500

ACATGGTACTCAAAAGATGTGGTTTGTGAGACAAATAATAGCACA

AATCAGACCCAGTGTTACATGAACCATTGCAACACATCAGTCATC
                600

ACAGAATCATGTGACAAGCACTATTGGGATGCTATAAGGTTTAGA

TACTGTGCACCACCGGGTTATGCCCTATTAAGATGTAATGATACC
                        700

AATTATTCAGGCTTTGCACCCAACTGTTCTAAAGTAGTAGCTTCT

```
ACATGCACCAGGATGATGGAAACGCAAACTTCCACATGGTTTGGC
                                         800

TTTAATGGCACTAGAGCAGAGAATAGAACATATATCTATTGGCAT


GGCAGAGATAATAGAACTATCATCAGCTTAAACAAATATTATAAT
                                         900

CTCAGTTTGCATTGTAAGAGGCCAGGGAATAAGACAGTGAAACAA


ATAATGCTTATGTCAGGACATGTGTTTCACTCCCACTACCAGCCG


ATCAATAAAAGACCCAGACAAGCATGGTGCTGGTTCAAAGGCAAA
   1000


TGGAAAGACGCCATGCAGGAGGTGAAGACCCTTGCAAAACATCCC


AGGTATAGAGGAACCAATGACACAAGGAATATTAGCTTTGCAGCG
            1100


CCAGGAAAAGGCTCAGACCCAGAAGTAGCATACATGTGGACTAAC


TGCAGAGGAGAGTTTCTCTACTGCAACATGACTTGGTTCCTCAAT
                        1200


TGGATAGAGAATAAGACACACCGCAATTATGCACCGTGCCATATA


AAGCAAATAATTAACACATGGCATAAGGTAGGGAGAAATGTATAT
                                        1300


TTGCCTCCCAGGGAAGGGGAGCTGTCCTGCAACTCAACAGTAACC


AGCATAATTGCTAACATTGACTGGCAAAACAATAATCAGACAAAC


ATTACCTTTAGTGCAGAGGTGGCAGAACTATACAGATTGGAGTTG
   1400


GGAGATTATAAATTGGTAGAAATAACACCAATTGGCTTCGCACCT
```

ACAAAAGAAAAAAGATACTCCTCTGCTCACGGGAGACATACAAGA
                    1500

GGTGTGTTCGTGCTAGGGTTCTTGGGTTTTCTCGCAACAGCAGGT

TCTGCAATGGGCGCTCGAGCGTCCCTGACCGTGTCGGCTCAGTCC
                         1600

CGGACTTTACTGGCCGGGATAGTGCAGCAACAGCAACAGCTGTTG

GACGTGGTCAAGAGACAACAAGAACTGTTGCGACTGACCGTCTGG
                              1700

GGAACGAAAAACCTCCAGGCAAGAGTCACTGCTATAGAGAAGTAC

CTACAGGACCAGGCGCGGCTAAATTCATGGGGATGTGCGTTTAGA
                                        1800

CAAGTCTGCCACACTACTGTACCATGGGTTAATGATTCCTTAGCA

CCTGACTGGGACAATATGACGTGGCAGGAATGGGAAAAACAAGTC

CGCTACCTGGAGGCAAATATCAGTAAAAGTTTAGAACAGGCACAA
          1900

ATTCAGCAAGAGAAAAATATGTATGAACTACAAAAATTAAATAGC

TGGGATATTTTTGGCAATTGGTTTGACTTAACCTCCTGGGTCAAG
                    2000

TATATTCAATATGGAGTGCTTATAATAGTAGCAGTAATAGCTTTA

AGAATAGTGATATATGTAGTACAAATGTTAAGTAGGCTTAGAAAG
                              2100

GGCTATAGGCCTGTTTTCTCTTCCCCCCCCGGTTATATCCAACAG

```
ATCCATATCCACAAGGACCGGGGACAGCCAGCCAACGAAGAAACA
                                        2200

GAAGAAGACGGTGGAAGCAACGGTGGAGACAGATACTGGCCCTGG


GCGATAGCATATATACATTTCCTGATCCGCCAGCTGATTCGCCTC


TTGACCAGACTATACAGCATCTGCAGGGACTTACTATCCAGGAGC
   2300

TTCCTGACCCTCCAACTCATCTACCAGAATCTCAGAGACTGGCTG


AGACTTAGAACAGCCTTCTTGCAATATGGGTGCGAGTGGATCCAA
        2400


GAAGCATTCCAGGCCGCCGCGAGGGCTACAAGAGAGACTCTTGCG


GGCGCGTGCAGGGGCTTGTGGAGGGTATTGGAACGAATCGGGAGG
                2500

GGAATACTCGCGGTTCCAAGAAGGATCAGACAGGGAGCAGAAATC


GCCCTCCTGTGAGGGACGGCAGTATCAGCAGGGAGACTTTATGAA
                        2600

TACTCCATGGAAGGACCCAGCAGCAGAAAGGGAGAAAAATTTGTA


CAGGCAACAAAATATGGA
```

7 - Rétrovirus selon l'une quelconque des revendications 1 à 6, caractérisé en ce que, pratiquement, son ARN n'hybride, ni avec le gène env et le LTR qui le jouxte, plus particulièrement avec la séquence de nucléotides 5290-9130, de HIV-1, ni avec des séquences de la région pol du génome de HIV-1, en particulier avec la séquence de nucléotides 2170-2240.

8 - Composition contenant au moins un antigène, notamment une protéine ou une glycoprotéine du rétrovirus HIV-2, selon l'une quelconque des revendications 1 à 7.

9 - Composition selon la revendication 8, caractérisée en ce qu'elle consiste en un extrait total ou en un lysat du susdit rétrovirus.

10 - Composition selon la revendication 8, caractérisée en ce que l'antigène consiste en au moins l'une des protéines internes du noyau (core) du susdit virus, notamment p12, p16 et p26, ayant respectivement des poids moléculaires apparents de l'ordre de 12.000, 16.000 et 26.000.

11 - Composition selon la revendication 8, caractérisée en ce qu'elle contient une glycoprotéine, gp140 ayant un poids moléculaire apparent de l'ordre de 130.000 à 140.000.

12 - Antigène qui ne produit qu'une seule bande en électrophorèse sur gel de polyacrylamide, caractérisé en ce qu'il comporte, en commun avec l'un des antigènes purifiés du rétrovirus HIV-2, un épitope reconnu par le sérum d'un porteur d'anticorps contre le virus HIV-2.

13 - Antigène purifié ayant les caractéristiques immunologiques de l'une des protéines ou glycoprotéines suivantes de HIV-2 : p12, p16, p26, p36, p42 et gp140.

14 - Antigène selon la revendication 13 ayant la séquence d'aminoacides ci-après ou partie de cette séquence dès lors qu'elle est reconnue par des anticorps anti-p12 :

```
                                    ArgLysAlaPheLys


      CysTrpAsnCysGlyLysGluGlyHisSerAlaArgGlnCysArg

                              1200
      AlaProArgArgGlnGlyCysTrpLysCysGlyLysProGlyHis


      IleMetThrAsnCysProAspArgGlnAlaGlyPheLeuGlyLeu

                                    1300
      GlyProTrpGlyLysLysProArgAsnPheProValAlaGlnVal


      ProGlnGlyLeuThrProThrAlaProProValAspProAlaVal


      AspLeuLeuGluLysTyrMetGlnGlnGlyLysArgGlnArgGlu

       1400
      GlnArgGluArgProTyrLysGluValThrGluAspLeuLeuHis


      LeuGluGlnGlyGluThrProTyrArgGluProProThrGluAsp

                    1500
      LeuLeuHisLeuAsnSerLeuPheGlyLysAspGln
```

15 - Antigène selon la revendication 13 ayant la séquence d'aminoacides ci-après ou une partie de cette séquence, dès lors qu'elle est reconnue par des anticorps anti-p16 :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

16 - Antigène selon la revendication 13 ayant la séquence d'aminoacides ci-après ou une partie de cette séquence, dès lors qu'elle est reconnue par des anticorps anti-p26 :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

17 - Antigène selon la revendication 13 ayant la séquence d'aminoacides ci-après, dès lors qu'elle est reconnue par des anticorps anti-gp140 :

ENVRN

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

41

```
ThrLysGluLysArgTyrSerSerAlaHisGlyArgHisThrArg
                    1500
GlyValPheValLeuGlyPheLeuGlyPheLeuAlaThrAlaGly

SerAlaMetGlyAlaArgAlaSerLeuThrValSerAlaGlnSer
                    1600
ArgThrLeuLeuAlaGlyIleValGlnGlnGlnGlnGlnLeuLeu

AspValValLysArgGlnGlnGluLeuLeuArgLeuThrValTrp
                    1700
GlyThrLysAsnLeuGlnAlaArgValThrAlaIleGluLysTyr

LeuGlnAspGlnAlaArgLeuAsnSerTrpGlyCysAlaPheArg
                    1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln
1900
IleGlnGlnGluLysAsnMetTyrGluLeuGlnLysLeuAsnSer

TrpAspIlePheGlyAsnTrpPheAspLeuThrSerTrpValLys
                    2000
TyrIleGlnTyrGlyValLeuIleIleValAlaValIleAlaLeu

ArgIleValIleTyrValValGlnMetLeuSerArgLeuArgLys
                    2100
GlyTyrArgProValPheSerSerProProGlyTyrIleGlnGln
```

```
IleHisIleHisLysAspArgGlyGlnProAlaAsnGluGluThr

                                        2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

           2400
GluAlaPheGlnAlaAlaAlaArgAlaThrArgGluThrLeuAla

GlyAlaCysArgGlyLeuTrpArgValLeuGluArgIleGlyArg

                 2500
GlyIleLeuAlaValProArgArgIleArgGlnGlyAlaGluIle

AlaLeuLeu***GlyThrAlaValSerAlaGlyArgLeuTyrGlu

                            2600
TyrSerMetGluGlyProSerSerArgLysGlyGluLysPheVal

GlnAlaThrLysTyrGly
```

18 - Méthode pour la détection in vitro de la présence d'anticorps anti-HIV-2 dans un liquide biologique, tel qu'un sérum, et plus particulièrement pour le diagnostic in vitro d'un SLA ou SIDA potentiel ou existant dû à un rétrovirus du type HIV-2, caractérisé en ce que l'on met en contact un sérum ou un autre milieu biologique provenant de la personne faisant l'objet du diagnostic, avec une composition selon l'une quelconque des revendications 8 à 11 ou avec un antigène selon l'une quelconque des revendications 12 à 17, et en ce que l'on détecte le conjugué immunologique éventuellement formé entre ces anticorps anti-HIV-2 et l'antigène ou les antigènes mis en oeuvre.

19 - Méthode selon la revendication 18, caractérisé en ce que l'on réalise la détection dudit conjugué immunologique en faisant réagir le conjugué immunologique éventuellement formé avec un réactif marqué formé soit par des anticorps anti-immunoglobulines humaines, soit par une protéine A bactérienne et en ce que l'on détecte le complexe formé entre le réactif et le susdit conjugué immunologique.

20 - Nécessaire ou kit pour la détection d'anticorps anti-HIV-2 dans un fluide biologique, notamment d'un porteur éventuel de ces anticorps, caractérisé en ce qu'il comprend :
- une composition telle que définie dans l'une quelconque des revendications 8 à 11 ou un antigène tel que défini dans l'une quelconque de revendications 12 à 17, et
- des moyens pour détecteur le conjugué immunologique résultant de la réaction immunologique entre l'antigène et ledit fluide biologique.

21 - Nécessaire ou kit selon la revendication 21, caractérisé en ce que les moyens pour détecter le conjugué immunologique formé comprennent des anti-immunoglobulines humaines ou une protéine A et des moyens pour détecter le complexe formé entre les anticorps anti-HIV-2 contenus dans le conjugué immunologique détecté.

22 - Composition immunogène contenant une glycoprotéine d'enveloppe du rétrovirus HIV-2, telle que la gp140 de ce rétrovirus, ou une partie de cette glycoprotéine, en association avec un véhicule pharmaceutiquement acceptable, approprié à la constitution de vaccins contre HIV-2.

23 - Composition selon la revendication 22, caractérisée en ce qu'elle contient au moins une partie immunogène d'une glycoprotéine contenant une ossature protéique ayant la séquence suivante :

ENVRN

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

46

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

```
IleHisIleHisLysAspArgGlyGlnProAlaAsnGluGluThr

                                          2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

          2400
GluAlaPheGlnAlaAlaAlaArgAlaThrArgGluThrLeuAla

GlyAlaCysArgGlyLeuTrpArgValLeuGluArgIleGlyArg

                      2500
GlyIleLeuAlaValProArgArgIleArgGlnGlyAlaGluIle

AlaLeuLeu***GlyThrAlaValSerAlaGlyArgLeuTyrGlu

                              2600
TyrSerMetGluGlyProSerSerArgLysGlyGluLysPheVal

GlnAlaThrLysTyrGly
```

24 - Composition immunogène selon la revendication 22 ou la revendication 23, caractérisée en ce qu'elle est dosée en antigène de façon à permettre l'administration d'une dose unitaire de 10 à 500, notamment de 50 à 100 microgrammes par kilogramme de corps.

25 - Anticorps monoclonal caractérisé par sa capacité à reconnaître spécifiquement l'un des antigènes conformes à l'une quelconque des revendications 14 à 17.

26 - Les hybridomes secréteurs de l'anticorps monoclonal selon la revendication 25.

27 - Acide nucléique, le cas échéant marqué, dérivé d'une partie au moins de l'ARN du virus HIV-2 ou de l'un de ses variants.

28 - Acide nucléique selon la revendication 27, contenant une partie au moins de l'ADNc correspondant à l'ARN génomique entier d'un rétrovirus HIV-2.

29 - Acide nucléique selon la revendication 27, caractérisé en ce qu'il contient la séquence de nucléotides :

GTGGAAGGCGAGACTGAAAGCAAGAGGAATACCATTTAGTTAAAGGACAG
GAACAGCTATACTTGGTCAGGGCAGGAAGTAACTAACAGAAACAGCTGAG
ACTGCAGGGACTTTCCAGAAGGGGCTGTAACCAAGGGAGGGACATGGGAG
GAGCTGGTGGGGAACGCCTCATATTCTCTGTATAATATACCCGCTGCTTG
CATTGTACTTCAGTCGCTCTGCGGAGAGGCTGGCAGATTGAGCCCTGGAG
GATCTCTCCAGCACTAGACGGATGAGCCTGGGTGCCCTGCTAGACTCTCA
CCAGCACTTGGCCGGTGCTGGCAGACGGCCCCACGCTTGCCTGCTTAAAA
ACCTTCCTTAATAAAGCTGCAGTAGAAGCA

30 - Acide nucléique selon la revendication 27, caractérisé en ce qu'il contient une séquence nucléotidique codant pour une partie au moins de la séquence d'aminoacides indiquée ci-après :

GAGRODN

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

706
ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGlnArgLysAlaPheLys

CysTrpAsnCysGlyLysGluGlyHisSerAlaArgGlnCysArg

AlaProArgArgGlnGlyCysTrpLysCysGlyLysProGlyHis

IleMetThrAsnCysProAspArgGlnAlaGlyPheLeuGlyLeu

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis

LeuGluGlnGlyGluThrProTyrArgGluProProThrGluAsp

LeuLeuHisLeuAsnSerLeuPheGlyLysAspGln

31 - Acide nucléique selon la revendication 27, caractérisé en ce qu'il contient une séquence nucléotidique codant pour une partie au moins de la séquence d'aminoacides indiquée ci-après :

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

32 - Acide nucléique selon la revendication 27, caractérisé en ce qu'il contient une séquence nucléotidique codant pour une partie au moins de la séquence d'aminoacides indiquée ci-après :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

33 - Acide nucléique selon la revendication 27, caractérisé en ce qu'il contient une séquence nucléotidique codant pour une partie au moins de la séquence d'aminoacides indiquée ci-après :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

706
ThrSerThrValGluGluGlnIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

34 - Acide nucléique selon la revendication 27, caractérisé en ce qu'il contient une séquence nucléotidique codant pour une partie au moins de la séquence d'aminoacides indiquée ci-après :

ENVRN

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

56

ThrCysThrArgMetMetGluThrGlnThrSerThrTrpPheGly

PheAsnGlyThrArgAlaGluAsnArgThrTyrIleTyrTrpHis

800

GlyArgAspAsnArgThrIleIleSerLeuAsnLysTyrTyrAsn

900

LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000

TrpLysAspAlaMetGlnGluValLysThrLeuAlaLysHisPro

ArgTyrArgGlyThrAsnAspThrArgAsnIleSerPheAlaAla

1100

ProGlyLysGlySerAspProGluValAlaTyrMetTrpThrAsn

CysArgGlyGluPheLeuTyrCysAsnMetThrTrpPheLeuAsn

1200

TrpIleGluAsnLysThrHisArgAsnTyrAlaProCysHisIle

LysGlnIleIleAsnThrTrpHisLysValGlyArgAsnValTyr

1300

LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400

GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

58

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

2400
GluAlaPheGlnAlaAlaAlaArgAlaThrArgGluThrLeuAla

GlyAlaCysArgGlyLeuTrpArgValLeuGluArgIleGlyArg

2500
GlyIleLeuAlaValProArgArgIleArgGlnGlyAlaGluIle

AlaLeuLeu***GlyThrAlaValSerAlaGlyArgLeuTyrGlu

2600
TyrSerMetGluGlyProSerSerArgLysGlyGluLysPheVal

GlnAlaThrLysTyrGly

35 - Acide nucléique selon l'une quelconque des revendications 28 à 34, caractérisé en ce qu'il est formé d'un acide nucléique recombinant, comprenant un acide nucléique provenant d'un vecteur, dans lequel le susdit ADNc ou partie d'ADNc se trouve inséré.

36 - Acide nucléique recombinant selon la revendication 35, caractérisé en ce qu'il est marqué.

37 - Procédé de détection d'un rétrovirus HIV-2 ou de son ARN dans un liquide ou tissu biologique, notamment en vue du diagnostic in vitro chez l'homme de la potentialité ou de l'existence d'un SLA ou d'un SIDA, caractérisé par la mise en contact des acides nucléiques contenus dans ce liquide ou tissu biologique avec une sonde contenant un acide nucléique selon l'une quelconque des revendications 28 à 36 dans des conditions d'hybridation stringentes, pendant le temps nécessaire à la réalisation de cette hybridation, le lavage de l'hybride formé, avec une solution assurance la conservation de ces conditions stringentes, et la détection de l'hybride formé.

38 - Procédé de production d'un rétrovirus HIV-2, caractérisé par la culture dans des lymphocytes T4 humains ou dans des lignées cellulaires permanentes dérivées de lymphocytes T4 et portant le phénotype T4, ces lymphocytes ou ces lignées ayant préalablement été infectés avec un isolat de virus HIV-2, et notamment lorsque le niveau d'activité de la transcriptase inverse (ou reverse-transcriptase) a atteint un seuil déterminé, par la récupération et la purification de quantités de virus libérées dans le milieu de culture de ces lymphocytes ou lignées, notamment par centrifugation différentielle dans un gradient de sucrose ou de métrizamide.

39 - Procédé pour la production d'antigènes spécifiques de HIV-2, rétrovirus caractérisé par la lyse, notamment à l'aide de détergents, tels que le SDS (par exemple SDS à 0,1 % dans un tampon RIPA) et la récupération du lysat contenant lesdits antigènes.

40 - Procédé de fabrication de l'une des protéines sus-définies (p12, p16 ou p26) ou d'une protéine ayant la structure de gp140, ou de parties déterminées de ces protéines, ce procédé étant caractérisé par l'insertion de la séquence d'acide nucléique correspondante dans un vecteur susceptible de transformer un hôte cellulaire choisi et de permettre l'expression d'un insérat contenu dans ce vecteur, par la transformation de cet hôte choisi par ledit vecteur contenant ladite séquence nucléique, par la culture de l'hôte cellulaire transformé et par la récupération et la purification de la protéine exprimée.

41 - Procédé de production d'une sonde d'hybridation pour la détection d'un ARN de rétrovirus HIV-2, caractérisé par l'insertion d'une séquence d'ADN, notamment selon l'une quelconque des revendications 27 à 35 dans un vecteur de clonage par recombinaison in vitro, par le clonage du vecteur modifié obtenu dans un hôte cellulaire compétent et par la récupération des ADN-recombinants obtenus.

0269520

FIG.1

FIG.2

FIG.3

0269520

CARTE DE RESTRICTION

FIG.4

0269520

FIG.5

# FIG.6

```
        10        20        30        40        50        60        70        80        90       100
GT GGAAGGCGAGACTGAAAGCAAGAGGAATACCATTTAGTTAAAGGACAGGAACAGCTATACTTGGTCAGGGCAGGAAGTAACTAACAGAAACAGCTGAG
                MNLI                                    ALUI                    MAEIII          PVUII
                                                                                                ALUI
                                                                                                   DDEI


       110       120       130       140       150       160       170       180       190       200
ACTGCAGGGACTTTCCAGAAGGGGCTGTAACCAAGGGAGGGACATGGAGGAGCTGGTGGGGAACGCCTCATATTCTCTGTATAATATACCCGCTGCTTG
PSTI                      MAEIII    MNLI  NLAIII   ALUI              MNLI                      BBVI
                               STYI              MNLI                                         PHU4NI
                                                                                                TN11111II


       210       220       230       240       250       260       270       280       290       300
CATTGTACTTCAGTCGCTCTGCGGAGAGGCTGGCAGATTGAGCCCTGGAGGATCTCTCCAGCACTAGACGGATGAGCCTGGGTGCCCTGCTAGACTCTCA
     RSAI                   MNLI         BANII   MNLI              MAEI      APYI BSP1286 MAEI    HPHI
                                         BSP1286 XMNI                        BSTNI         HINFI
                                         APYI    DPNI                        ECORII
                                         BSTNI   MBOI                        SCRFI
                                         ECORII  NDEII                          BANI
                                         SCRFI   SAU3IIIA


       310       320       330       340       350       360       370       380
CCAGCACTTGGCCGGTGCTGGCAGACGGCCCCACGCTTGCCTGCTTAAAAACCTTCCTTAATAAAGCTGCAGTAGAAGCA
        MAEIII           MAEIII                              ALUI
         HAPII           SAU96A                              BBVI
         NHAII                                               PHU4NI
         MSPI
```

FIG.7

gag    pol    t    env    F

FIG.7A

HIV-1 sonde

HIV-2 ADNc(E2)

Pv  Pv  (A)n

1 kb

B Ps  Ps

FIG.7B

B

```
                              PvuI              PstI
HIV.2  AGTAACTAACAGAA------ACAGCTGAGACTGC----AGGGACTTTCCAGAAGGGGCTG
       ::: :: :  ::::  :::::   :   :: ::  ::::::::::::  :: ::::::
HIV.1  AGT-ACTTCAAGAACTGCTGACATC-GAGCTTGCTACAAGGGACTTTCCGCTGGGGACTT
           9000        9010        9020        9030        9040

HIV.2  TAACCAA-------------GGGAGGGACATGGGAG----GAGCTGGTGGGGAACGCCTC
       : :::       ::: ::::::::: ::::    ::::
HIV.1  T--CCAGGGAGGCGTGGCCTGGGCGGGACTGGGGAGTGGCGAGC-----------CCTC
           9050        9060        9070        9080        9090

HIV.2  ATATTCTCTG[TATAAA]ATACCCGCTGCTTGCATTGTACTTCAGTCGCTCTGCGGAGAGG
       ::: :    :::::  :: ::::: :::::  ::: :::::  :: ::::::::
HIV.1  AGATG--CTGCA[ATAAG]CAGCTGCTTTTTGCC-TGTACTGG-GTCTCTCTGGTTAGAC-
           9100        9110        9120        9130        9140
                                                      U3 R

HIV.2  CTGGCAGATTGAGCCCTGGGAGGTTCTCTCCAGCACTAGCAGGTAGAGCCTGGGTGTCCC
       :::::: ::   :::::::: ::: :::: :::::  :::::
HIV.1  -----CAGATTTGAGCCTGGGAGC-TCTCTGGCTAACTAGGGAACCCAC-----------
           9150        9160        9170        9180        9190

HIV.2  TGCTAGACTCTCACCAGCACTTGGCCGGTGCTGGGCAGACGGCCCCACGCTTGCTTGCTT
                                                           :::::::
HIV.1  --------------------------------------------------------TGCTT

HIV.2  AAAAACCTCCTT[AATAAA]GCT-GCC---AGTAGAAGCA
       :::::   :::: :::::::  :::  :::   :
HIV.1  AAG-----CCT[AATAAA]GCTTGCCTTGAGTGCTTCAA
               9210        9220
                  HindIII              R
```

FIG.8

0269520

SONDE HIV-2-   1   2   3   4   5   6   7   8   9   10   11   12

Tm-42   •   •   •       •               •   •   •

Tm-20   •   •   •       •                   •   •

Tm-3                                            •

SONDE HIV-1

Tm-3   •   •   •   •   •   •   •   •   •   •   •   •

# FIG.9